(19) Europäisches Patentamt / European Patent Office / Office européen des brevets

(11) **EP 3 479 671 B1**

(12) **FASCICULE DE BREVET EUROPEEN**

(45) Date de publication et mention
de la délivrance du brevet:
**06.01.2021   Bulletin 2021/01**

(51) Int Cl.:
***A01C 21/00*** *(2006.01)*      ***G01N 33/24*** *(2006.01)*

(21) Numéro de dépôt: **18203377.9**

(22) Date de dépôt: **30.10.2018**

(54) **MÉTHODE POUR LA FORMATION D'UN INDICATEUR ÉLÉMENTAIRE DE L'EFFICACITÉ AGRONOMIQUE D'AZOTOBACTÉRIES EN SOLS ARABLES**

METHODE ZUR BILDUNG EINES ELEMENTAREN INDIKATORS FÜR DIE AGRONOMISCHE WIRKSAMKEIT VON STICKSTOFFBAKTERIEN IN PFLÜGBAREN BÖDEN

METHOD FOR FORMING AN ELEMENTARY INDICATOR OF AGRONOMIC EFFICIENCY OF AZOTOBACTER IN ARABLE SOILS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **02.11.2017   FR 1771159**

(43) Date de publication de la demande:
**08.05.2019   Bulletin 2019/19**

(73) Titulaire: **Polyor SARL**
**54000 Nancy (FR)**

(72) Inventeur: **CLAUDE, Pierre-Philippe**
**54000 Nancy (FR)**

(56) Documents cités:
**EP-B1- 2 819 498          DE-T2- 60 037 334**
**US-A1- 2013 046 468          US-B2- 8 962 336**

**Description**

**DOMAINE TECHNIQUE DE L'INVENTION**

Agronomie, y compris le pilotage de la fertilisation azotée raisonnée (FR) des grandes cultures par échantillonnage ou prélèvement de sols arables. Il est aussi question de bactéries et d'azotobactéries en proximité de résidus de culture cellulosiques au sol (RCS).

**ÉTAT DE LA TECHNIQUE**

*1. Fertilisation raisonnée (FR) et fertilisation azotobactérienne raisonnée (FAR)*

Bien qu'un consensus existe autour de la méthode Comifer (2013) du bilan, ce calcul des doses prévisionnelles d'engrais azoté (dX) est aujourd'hui controversé (Ravier et al. 2015, 2016, 2017). Plus particulièrement, la *fertilisation raisonnée* (FR) au sens du Comifer ne comptabilise pas véritablement l'activité des azotobactéries telluriques, notamment en présence de *résidus de culture (cellulosiques) au sol* (RCS). En effet, ce bilan estime que les gains d'N par *fixation non symbiotique* ($F_{ns}$) ne font que compenser les pertes gazeuses d'N par *dénitrification* ($G_s$), et que globalement le bilan agronomique de la Fns est en ce sens nul ou négligeable. Enfants pauvres de la microbiologie des sols, ces azotobactéries non-symbiotiques ont longtemps été confinées à la famille *Azotobacteracea.* Or, nous savons aujourd'hui que le potentiel azotobactérien d'un sol en présence de RCS est bien supérieur aux quelques kg-N/ha/an rapportés. Cette sous-estimation du potentiel azotobactérien peut expliquer pourquoi la biofertilisation azotobactérienne par inoculation des RCS (Claude et Fillion 2004) n'est toujours pas aussi répandue qu'espéré. L'intégration de l'activité de ces azotobactéries dans le calcul des doses prévisionnelles d'engrais N, et P, (cf. FAR, *fertilisation azotobactérienne* azotée ; EP17196251.7) nécessite le développement *d'indices élémentaires* spécifiques. De plus, l'activation de cette *zone d'activité résidusphérique* (ZAR), y compris par inoculation azotobactérienne des RCS (Claude et Fillion 2004 ; FR2833016, EP2314669, EP2684588, etc.), pourra aussi affecter ce calcul. D'autres états de la technique sont US 2013/046468 A1 et EP 2819498 A1.

*2. Résidus de d'azote minéral (Nm), post-récolte, automnaux et sortie-hiver*

Les grandes cultures agronomiques mobilisent qu'une partie de l'azote minéral (Nm) libéré du sol et/ou apporté par fertilisation. Les reliquats d'azote qui en résultent sont aujourd'hui estimés ponctuellement par échantillonnage, soit à l'automne (RAT), soit - le plus souvent, en sortie hiver (RSH ; Comifer 2013). Ces RSH seraient ainsi disponibles à la culture suivante et réduiraient d'autant dX. Or, ces mesures - RSH surtout, sont notoirement imprécises pour diverses raisons, y compris la grande variabilité spatiale sur des distances aussi courtes que 10 m (Tabor et al. 1985, Cambardella et al. 1985). L'utilisation de valeurs de densités apparentes de la couche arable, arbitraires et/ou imprécises le plus souvent, n'arrange rien. De plus, les RAT, par définition sur 30 cm, sont lessivés en profondeur pendant l'interculture. Or, l'importance de cette lixiviation ne peut pas être déterminée ponctuellement sur une parcelle agronomique lors de la simple détermination des RSH superficielles. Enfin, il y a les coûts et une logistique complexe puisque la détermination des RSH nécessite l'envoi des échantillons dès la sortie hiver, période très chargée pour l'agriculteur, dans l'espoir d'obtenir un résultat du laboratoire dès que possible. C'est pourquoi les RAT sont été proposés comme variable de choix au lieu des RSH (EP2942621). Cela dit, cette invention demeure imparfaite et d'autres indices élémentaire d'eAZB sont recherchés.

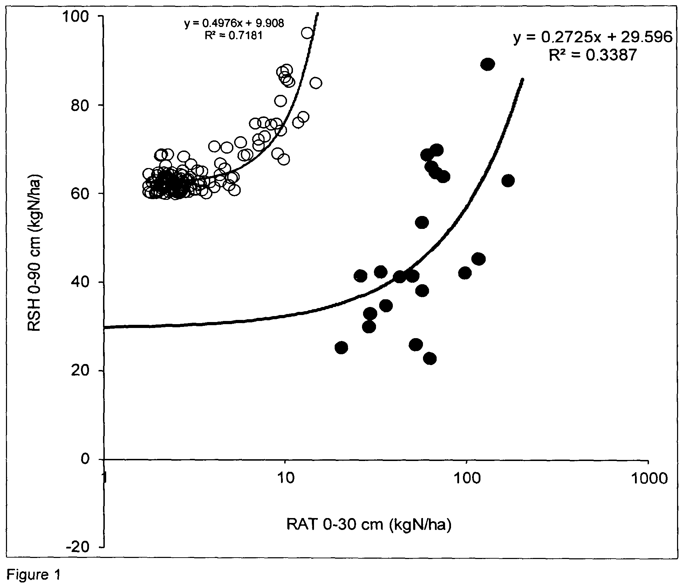

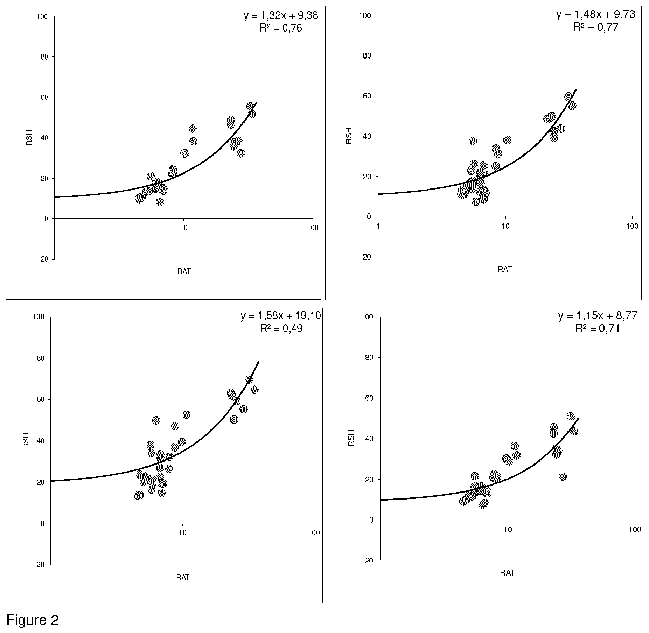

eAZB appréciable, imprécisément, via les RSH lorsque RAT = 0 à la Figures 1. Ces RSH de l'ordre de 10 à 30 uN (Nb. 1 uN = 1 kg-N/ha) sont commensurables avec les recommandations d'engrais N supplémentaires (20 uN) préconisées par la méthode du bilan Comifer (2013) en présence de RCS. On peut s'étonner de cette recommandation du fait que les RCS semblent plutôt apporter de l'Nm en sortie hiver. En effet, à la Figure 2, les RSH lorsque RAT = 0 en présence de RCS selon une série de modélisation Stics™ (Brisson et al. 2008) sont eux aussi de l'ordre de 10 à 20 uN. Il est vraisemblable que le l'immobilisation transitoire d'Nm profitent aux azotobactéries du sol - très sujettes à rétroaction négative par l'Nm, d'où in fine une contribution nette. Le renforcement du potentiel azotobactérien du sol, y compris par inoculation des RCS (Claude et Fillion 2004) augmentera nécessairement cette contribution en N des azotobactéries résidusphériques (alias « pAZB »). Or, pAZB n'est pas actuellement intégrée dans le calcul de la dX faute de suffisamment *d'indices élémentaires* satisfaisant du potentiel azotobactérien du sol et/ou de l'efficacité de l'azotobactérisation des RCS (alias « eAZB »).

*3. Échantillonnage versus prélèvement des parcelles agricoles*

Déterminer RAT et/ou RSH selon le Comifer 2013 nécessite l'échantillonnage de la zone d'enracinement. Or, l'étendue de ces zones n'est pas connue ponctuellement, l'homme du métier doit échantillonner une profondeur donnée, disons 60 cm au plus dans la plupart des cas, profondeur toute aussi arbitraire que les susdites densités apparentes. Ce problème d'échantillonnage fait que les susdits RAT et RSH sont donc d'assez pauvres prédicteurs du pouvoir de fourniture en azote du sol. Cela résulte, il me semble, d'un mauvais usage des notions *d'échantillonnage* et de *prélèvement*, voire de la confusion des genres. Un échantillon consiste en un ensemble représentatif d'une « population mère » possédant les mêmes caractéristiques constituées soit au hasard, soit suivant une quelconque méthode systématique. Or, trop souvent, seul les teneurs élémentaires (mg/kg de N, P, K, etc.) sont mesurées avec précision, et non le volume de sol et l'étendue de la zone d'enracinement ; l'échantillonnage précis et routinier de la parcelle est donc illusoire. Le prélèvement, lui, consiste plus simplement en une certaine portion sur un total ou sur une masse, sans prétentions statistiques et/ou de représentativité. Le prélèvement n'est pas entiché des imprécisions et impertinences trop souvent associés à l'échantillonnage de sols spatialement très variables ; le prélèvement peut par contre, vue sa taille réduite, être échantillonné précisément.

Sigles et définitions

**Azotobactérisation** : renforcement, y compris par inoculation (Claude et Fillion 2004), des populations d'azotobactéries telluriques en proximité des résidus de culture au sol (RCS) et de cipans (cultures intermédiaires pièges à nitrates ; Claude et al. 1993 ; Inra 2013).

**eAZB™** : efficacité de l'azotobactérisation des RCS en termes agronomiques (rendements en grains ou en protéines) par rapport à un témoin avec RCS non-azotobactérisés.

**Échantillonnage pondéral** (ep) : processus pour l'obtention de mesures de volume, de densité et de teneur d'une zone bien défini d'un sol arable en surface de manière à estimer précisément la masse élémentaire (eg. N), par unité de poids tellurique (eg. mg/kg) ou de surface arable (kg/ha).

iAZB™ : indice agrégé d'eAZB

**Indicateurs agrégés** (cf. Lairez et al. 2015) : résultent de l'agrégation de plusieurs indicateurs élémentaires impliqués dans un même phénomène, ces indicateurs élémentaires étant exprimés dans une unité commune ou un score (Si) avant d'être agrégés. Cette dernière repose, soit sur une relation empirique démontrée, soit sur des conventions établies par des experts définissant ce qui compose un tel critère élémentaire. **Indicateurs élémentaires** (cf. Lairez et al. 2015) : utilisés quand un phénomène ne peut pas être décrit directement, ou précisément, en raison de sa complexité. Ils fournissent des informations sur un phénomène en le quantifiant ou le qualifiant. Ils simplifient l'information en se basant sur des observations, des données, des sorties de modèles, ou des mesures.

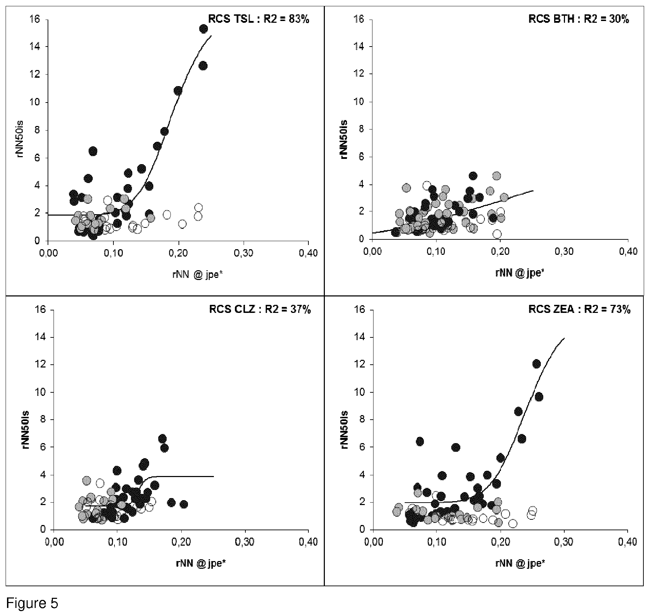

**jpe** (jours post enfouissement) : se rapportant à rNN50is, la période de temps écoulée entre l'enfouissement des RCS, généralement à l'automne et un quelconque prélèvement de la ZAR afin de déterminer ponctuellement rNN. Un cas particulier est **jpe***, moment auquel la relation rNN50is :: rNN est la plus appréciable en termes de corrélation ; à ce moment, cette relation est généralement sigmoïdale (logistique ; cf. équation 2) et permet ainsi d'identifier rNN50is le plus révélateur d'eAZB, i.e. l'efficacité agronomique de l'azotobactérisation des RCS (cf. Figure 5).

**jpi** (jours post initiation) : se rapportant à rNN50iv, la période de temps, en jours, écoulée entre le début et la fin de l'incubation in vitro du prélèvement de la ZAR.

**pAZB™** : contribution en azote des azotobactéries résidusphériques

**Prélèvement indiciel** (pi) : processus pour l'obtention d'une certaine portion sur un total ou sur une masse. Contrairement à l'échantillonnage, le prélèvement n'a pas à être priori statistiquement ou pondéralement représentatif de l'ensemble duquel il provient, mais peut lui-même faire l'objet d'un échantillonnage pondéral, cette fois-ci très précis cependant.

**RAT** : reliquats Nm automnaux obtenue par échantillonnage pondéral ; par définition, les RAT sont évalués ici sur 30 cm de profondeur, et cela à l'automne immédiatement avant ou après l'enfouissement des RCS.

**rNN** : ratio massique $N-NH_4/N-NO_3$ du sol et/ou de la solution du sol et/ou d'un prélèvement de la ZAR. Sans unités et ne nécessitant pas l'interprétation de la profondeur du sol et/ou de sa densité apparente afin d'exprimer Nm en kg-N/ha, cette variable est assez précise, et moins arbitraire ; du fait du métabolisme N des microorganismes dans le sol, il est aussi le reflète de l'activité immédiate de cette microflore tellurique.

**rNN50is** : rNN50 in situ. indice de fourniture d'Nm sortie d'hiver calculé sur la base du ratio massique $N-NH_4/N-NO_3$ du sol et/ou de la solution du sol à l'automne post récolte et en sortie hiver de manière à tenir compte de la lixiviation d'Nm en profondeur pendant l'interculture. Conceptuellement, il s'agit donc rapport RSH/RAT = rNN50is mais calculé à partir d'une relation empirique, avantageusement logistique avec asymptotes de type rNN50is = (a + (d -a) / [1 + (rNNjpe* / c)$^b$]), lorsque rNN = rNNjpe*, i.e. la valeur rNN lorsque **jpe** (jours post enfouissement) = **jpe*** (infra). Les paramètres a et d sont des paramètres d'asymptotes (minima et maxima, respectivement), b le paramètre de pente, et c l'abscisse au point de mi-pente (50%) dont l'ordonnée est (a+b)/2.

**rNN50iv** : rNN50 *in vitro*. ratio massique $N-NH_4/N-NO_3$ du sol et/ou de la solution du sol établi à partir d'un prélèvement automnal et/ou post récolte peu avant ou après l'enfouissement des RCS, ledit prélèvement ayant été préalablement incubé jusqu'à obtention d'une phase accélérée d'augmentation de rNN avec le temps d'incubation (jours post initiation, **jpi**) atteignant *50% du rNN maximum.* Ce point d'inflexion desdites accélérations est avantageusement établi à l'aide de fonctions logistiques, par exemple à quatre paramètres et de type rNN = a + (d -a) / [1 + (jpi / c)$^b$] sachant que les paramètres a et d sont des paramètres d'asymptotes (a étant le minimum et d le maximum), b le paramètre de pente, et surtout c correspond à l'abscisse du point de mi-pente dont l'ordonnée est (a+b)/2 lorsque rNN = rNN50iv.

**RSH** : reliquats d'azote minéral (Nm ; kg-N/ha, ou mg-N/kg-sol) sortie-hiver, avant le premier apport d'engrais N et obtenue par échantillonnage pondéral et comprenant l'ensemble de l'Nm, y compris celui provenant de la lixiviation, sur 90 à 120 cm de profondeur.

**ZAR** (zone activité résidusphérique) : volume de sol entourant les résidus de culture cellulosiques en cour de décomposition dans ou sur le sol et affectant l'activité des microflore microbiennes et dont l'étendue est généralement limitée à quelques mm de part et d'autre lesdits résidus de culture.

## DIVULGATION DE L'INVENTION

### Problème technique

Le problème technique est tripartite. Premièrement, la méthode du bilan (Comifer 2013) dépend d'une estimation des RSH imprécises du fait que les RAT, initialement confinés en surface de la couche arable, vont migrer en profondeur, plus ou moins rapidement selon le type de sol, la pluviométrie, etc. Deuxièmement, il y a confusion entre échantillonnage (pondéral) et prélèvement (indiciel). L'échantillon permet d'avoir une idée d'ensemble de la population de laquelle il provient en vue d'obtenir un résultat représentatif statistiquement significatif. Le prélèvement, lui, n'a pas de prétentions statistiques ou représentatives à l'égard des mesures quantitatives pondérales ; le prélèvement ne concerne qu'une certaine portion prise sur un total, ou sur une masse (poids). Enfin et troisièmement, la méthode du bilan du Comifer (2013) n'intègre pas la contribution de l'azotobactérisation des RCS, néglige pAZB et nuit ainsi au développement de la *fertilisation azotobactérienne raisonnée* (FAR).

### Solution technique

Je propose d'échantillonner ce prélèvement de terre caractéristique, mais pas nécessairement représentatif, de la parcelle agricole. La solution technique prévoit donc l'échantillonnage (pondéral) des rNN d'un prélèvement (indiciel) de terre. L'invention invoque donc les notions *d'échantillonnage pondéral*, ou massique, et de *prélèvement indiciel*, mais inverse l'ordre habituel dans lequel s'effectuent ces deux opérations. Le prélèvement concerne donc, non les habituelles couches arables et/ou zones d'enracinement, mais plutôt une certaine *zone d'activité résidusphérique* (ZAR). La solution technique s'appuie sur, non la masse d'Nm, mais sur le ratio rNN = [N-NH$_4$/N-NO$_3$]. En effet, rNN est synonyme d'une accumulation d'N-NH$_4$ à un moment donné ; un rNN plutôt élevé signifie que les ions NH$_4$ en voie de nitrification s'accumulent, vraisemblablement du fait d'une récente (immédiate) activité microbienne (azotobactérienne) plus importante qu'autrement sans RCS (azotobactérisés).

Concrètement, je propose une méthode pour la formation d'un indicateur élémentaire de l'efficacité de l'azotobactérisation (eAZB) des résidus de culture cellulosiques au sol (RCS) et de culture intermédiaires pièges à nitrates (cipan) applicable en fertilisation raisonnée (FR) par mesure du ratio N-NH$_4$/N-NO$_3$ (rNN) de l'azote minéral (Nm) du sol sans recourir à l'échantillonnage pondéral de la zone vadose ou d'enracinement et caractérisée en ce que la *zone d'activité résidusphérique* (ZAR), c'est-à-dire le volume de sol entourant les susdits résidus de culture en cour de décomposition dans ou sur le sol et affectant l'activité des microflores microbiennes, fait l'objet d'un simple *prélèvement* indiciel échantillonné pondéralement pour Nm et rNN, et en ce que ;

> les RCS sont azotobactérisés par renforcement, y compris via l'inoculation, des populations d'azotobactéries telluriques en proximité des RCS et des cipans, avant leur enfouissement et la constitution de la ZAR,

> la profondeur de la ZAR est égale à celle de l'enfouissement des RCS,

> la valeur critique de rNN, rNN50, est déterminée soit in vitro (rNN50iv), soit in situ (rNN50is), et représente la valeur médiane entre les valeurs rNN minimales et maximales respectives atteintes au cour de la minéralisation desdits RCS à travers le temps.

La ZAR est repérée visuellement par la présence manifeste de RCS à travers une coupe de la surface du sol, ou implicitement sur la base du réglage de l'instrument aratoire utilisé pour l'enfouissement des RCS tel que par exemple une charrue à socs ou encore des instruments aratoires à disques et dérivés de disques. La ZAR pour détermination de rNN50iv est <u>prélevée en automne</u> immédiatement après l'enfouissement des résidus de culture cellulosiques (RCS).

Ce prélèvement de la ZAR est échantillonné de manière répétée afin de déterminer rNN à travers le temps et d'en établir la cinétique, avantageusement journalière, par exemple à l'aide de microsondes spécifiques pour l'$N\text{-}NO_3$ et $N\text{-}NH_4$. Pour les RCS de types TSL (tournesol) et ZEA (maïs) enfouis tardivement à l'automne ou encore les RCS de types BTH (blé d'hiver, céréales) et CLZ (colza) enfouis hâtivement à la fin de l'été les prélèvements de la ZAR sont effectués entre 0 et 7 jpe (jours post enfouissement), et plus particulièrement dès que possible post enfouissement.

Le moment donné pour l'établissement de rNN correspond à une augmentation accélérée de rNN au cours de l'incubation in vitro dudit prélèvement de la ZAR à température et humidité constantes, et plus particulièrement au moment correspondant au point d'inflexion d'une fonction empirique avec plateaux asymptotiques de type :

$$rNN = a + (d\text{-}a) / [\, 1 + (jpi\,/\,c)^b\,]$$

sachant que les paramètres a et d sont des paramètres d'asymptotes (a étant le minimum et d le maximum), b est le paramètre de pente, le paramètre c correspond à l'abscisse du point de mi-pente, ou *point d'inflexion*, dont l'ordonnée est (a+b)/2 lorsque rNN = rNN50iv, et jpi (jours post initiation) la période de temps, en jours, écoulée entre le début et la fin de l'incubation in vitro du prélèvement de la ZAR. Il sera aussi possible, à terme, d'accélérer cette cinétique rNN de manière à rendre l'invention plus efficace, d'ajouter une certaine quantité de substrat carboné au microcosme comme le proposent EP2684588, EP2314669, ou encore EP2213154.

La ZAR pour détermination de rNN50is est <u>prélevée en sortie hiver</u>. Ce prélèvement de la ZAR est échantillonné ponctuellement de manière répétée afin d'obtenir une mesure représentative et ponctuelle de rNN dans ce prélèvement. L'indice rNN50is est ici obtenu directement d'une fonction empirique décrivant rNN50is selon la valeur de rNN au moment des susdits prélèvements (jpe*), jpe* étant le jpe auquel la relation rNN50is :: rNN est la plus forte, et plus particulièrement à l'aide d'une fonction logistique avec asymptotes à 4 paramètres de type;

$$rNN50 = a + (d\text{-}a) / [\, 1 + (rNN_{jpe^*}\,/\,c)^b\,] = rNN50is$$

sachant que a et d sont des paramètres d'asymptotes (a étant le minimum et d le maximum), b est le paramètre de pente, et c le paramètre correspondant à l'abscisse du point de mi-pente dont l'ordonnée est (a+b)/2 lorsque rNN = rNN50is. Enfin, le moment du prélèvement in situ de la ZAR, en nombre de jpe, le plus révélateur de rNN entant qu'indice de la contribution de Nm (jpe*) est situé, pour les RCS de types TSL et ZEA enfouis tardivement à l'automne, entre 50 et 70 jpe, et plus particulièrement à 60 jpe ; pour les RCS de types BTH et CLZ enfouis hâtivement à la fin de l'été, ce moment est situé entre 120 et 160 jpe, et plus particulièrement à environ 150 jpe.

Les valeurs rNN50iv et/ou rNN50is, étant elles corrélées à eAZB - i.e. efficacité de l'azotobactérisation des RCS en termes agronomiques (rendements en grains ou en protéines) par rapport à un témoin avec RCS non-azotobactérisés, sont utilisées entant qu'indice prédisant eAZB sur la culture suivante implantée sur ces RCS enfouis,

*Avantages apportés et activité inventive*

L'invention permet d'éviter de recourir impérativement à des mesures-pondérales de "azote minéral du sol (Nm), soit à l'automne (RAT), soit en sortie hiver (RSH). En effet, ces mesures foncièrement imprécises et donc souvent impertinentes supposent une appréciation correcte de la profondeur et de la densité apparente de la zone vadose, deux mesures difficilement obtenues avec précision par i'homme du métier de la cadre du conseil agronomique. Ne plus devoir effectuer, ou estimer, ces mesures massiques de Nm par *échantillonnage* - par opposition à un simple *prélèvement*, de la zone d'enracinement, est inhabituel. De plus, l'utilisation d'indices non pondéreux (eg. rNN, rNN50is et rNN50iv) plus précis que les habituelles mesures de RAT et RSH permet de contourner la méthode du bilan (Comifer 2013) au profit d'une véritable *fertilisation azotobactérienne raisonnée* (FAR ; Claude et Fillion 2004 ; Claude 2017 ; EP17196251.7) intégrant cette fois-ci la contribution de l'activité diazotrophique de la ZAR.

## BRÈVE DESCRIPTION DES DESSINS ET FIGURES

**Figure 1** : RSH (reliquats sortie hiver ; kg Nm/ha sur 90 cm) en fonction des RAT (reliquats automnaux d'Nm ; kg-N/ha sur 30 cm). Ces données de Mengel 1990, et Claude 1990 et Claude et al. 1993 (en abîme), illustrent qu'il est possible de prédire RSH ne connaissant que les RAT. Encore faut-il tenir compte du type de RCS (ici, colza et maïs), l'environnement pédo - climatique, etc. Les RSH en profondeur sont essentiellement du même ordre de grandeur que les RAT en surface du sol, et les RSH lorsque RAT = 0,00 est de 5 à 10 unités d'N (uN ; kg-N/ha). L'échelle logarithmique des RAT masque que la relation RSH :: RAT est imprécise lorsque les RAT ≤ ~30 uN.

**Figure 2** : Selon les modélisations effectuées à partir des données aux Tableaux 1 et 2, les RSH (sur 120 cm de profondeur) selon RAT (sur 30 cm de profondeur) pour quatre types de RCS, soit dans l'ordre habituelle de tournesol (TSL), blé tendre d'hiver (BTH), colza (CLZ) et maïs-grain (ZEA) ; les modélisations Stics décrites dans le texte. La progression des RSH, bien que quasi unitaire par rapport à RAT, implique encore une fois des RSH négatifs lorsque RAT = 0,00. A noter la séparation en deux groupe des RSH :: RAT selon l'importance des RAT (cf. Figure 1).

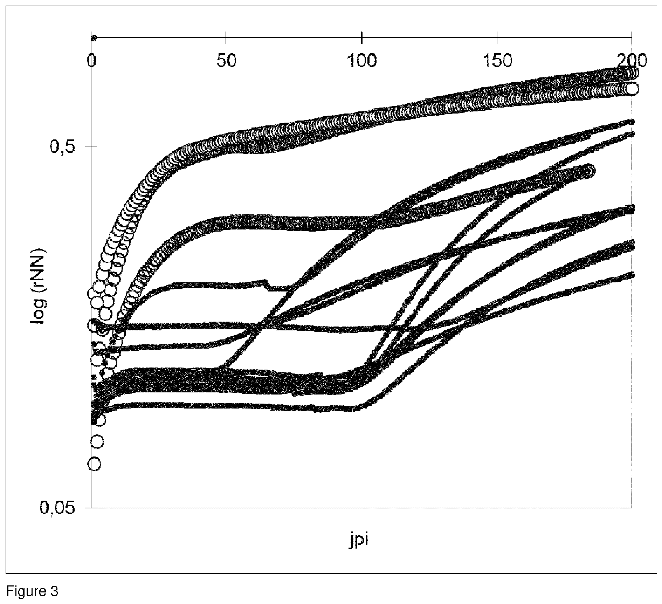

**Figure 3** : Augmentation de rNN selon le nombre de *jours post initiation* (jpi) de l'incubation in vitro pour les situations pédologiques aux Tableaux 2 et 3 à température et humidité constantes. Les bacs de prélèvement sont ici ceux proposés dans EP13366004.3. rNN (N-NO$_3$/N-NH$_4$) peut être suivi à travers le temps, par exemple à l'aide de micro-sondes (Bendikov et al. 2005, Yang et al. 2011, Larsen et al. 1996). Ces relations [rNN :: jpi] permettent de déterminer le rNN50iv, i.e. la valeur de rNN lorsque le jpi est égale au paramètre c pour ce type de fonctions logistiques avec asymptotes (eg. équation (1)).

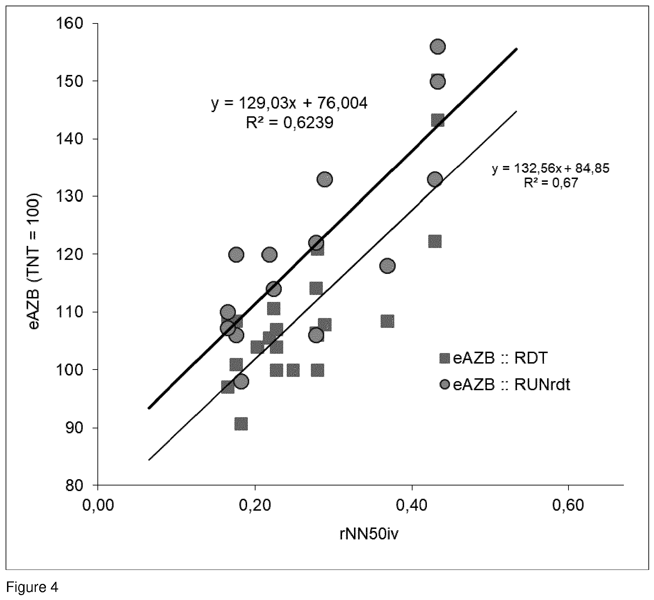

**Figure 4** : Relations empiriques décrivant l'efficacité de la fertilisation azotobactérienne des RCS (eAZB ; témoin sans azotobactérisation = TNT = 100) en fonction de rNN50iv (Tableau 2). Ces ratios des rendements agronomiques en grains (RDT) ou unitaires (RUNrdt ; unité de RDT par unité d'engrais N) avec et sans azotobactérisation (eAZB) progressent selon l'augmentation de rNN50iv.

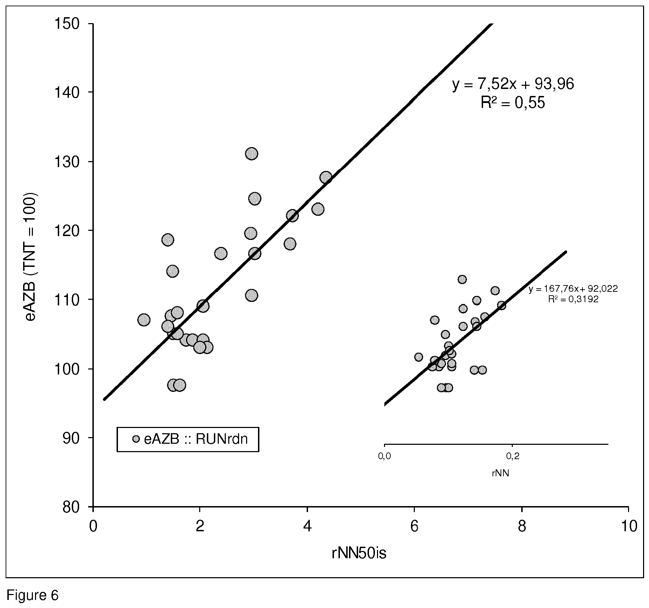

**Figure 5** : rNN50is selon la valeur de rNN à jpe* (rNN$_{jpe*}$). Ces relations empiriques issues des modélisation (Tableaux 3 et 4) sont ici avantageusement paramétrées (Tableau 5) à l'aide d'une fonction non linéaire *logistique* et asymptotique à quatre paramètres (équation 2). Les valeurs d'rNN50is ainsi calculées à partir de rNN$_{jpe*}$ seront mieux corrélées avec eAZB (Figure 6) qu'avec de simples rNN non transformé (Figure 6, en abîme).

**Figure 6** : eAZB (témoin = TNT = 100) en termes de rendement unitaire protéique du (RUNrdn ; kg de protéine récoltée par uN) du blé d'hiver (BTH) est bel et bien fonction positive d'rNN50is calculé logistiquement à partir de la régression non linéaire à quatre paramètres (équation 2), Tableaux 5, 6 et 7). Sans être parfaite, cette relation eAZB :: rNN50is (Tableau 7) est plus prononcée que la relation témoin eAZB :: rNN (en abîme).

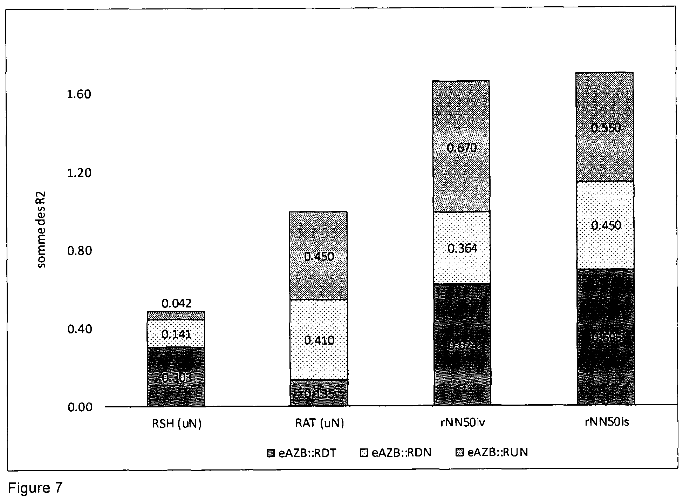

**Figure 7** : Les coefficients de détermination (R$^2$) pour eAZB en termes de RDT, RDN et RUN selon les RSH et RAT, d'une part, et selon les nouveaux indicateurs rNN50iv et ib au sens de l'invention, d'autre part. Puisqu'il s'agit de trois valeurs d'eAZB différentes, on peut additionner (superposer) ces valeurs de R$^2$.

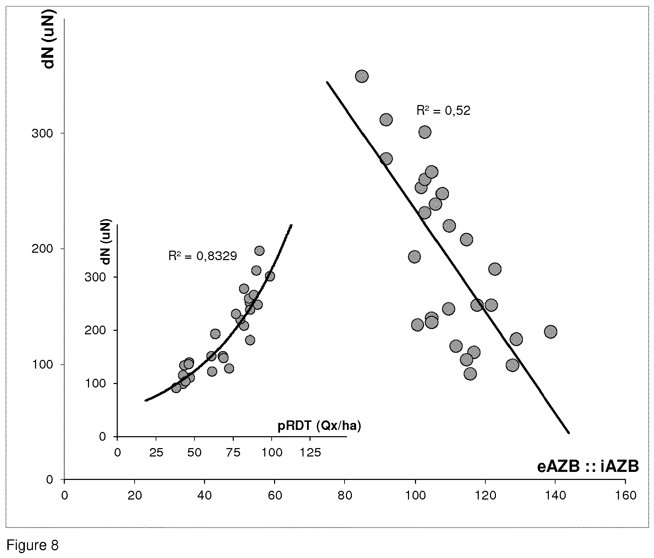

**Figure 8** : Application de l'invention en fertilisation raisonnée. Les valeurs rNN50iv et/ou rNN50is, étant elles corrélées à eAZB peuvent être utilisées entant qu'indice prédisant eAZB sur la culture suivante implantée sur ces RCS enfouis. Or, cet valeurs d'eAZB sert à calibrer la dose d'engrais N, dose dite ici « dN », alias dX selon la méthode du bilan maintenant dépassée. A partir de données agro-pédo-climatiques de 30 essais agronomiques de blés de de colza (2011 à 2015), des indices *élémentaires* d'eAZB tels que rNN50 sont agrégés par analyse multicritère en un indice *composé* d'eAZB, **iAZB**™ (EP17196251.7). **dN** (uN) ainsi calculée selon le potentiel de rendement escompté (**pRDT** ; Qx/ha) est dégressive selon eAZB :: iAZB en raison de la contribution des azotobactéries au régime azoté de la culture.

## MODE DE RÉALISATION PRÉFÉRÉ DE L'INVENTION

L'invention est réalisée (i) in vitro, et/ou (ii) in situ. Les valeurs de rNN50iv (in vitro) sont dans tous les cas corrélés à eAZB et peuvent servir à rationner dX dans le cadre d'une fertilisation raisonnée azotobactérienne. Idem pour les valeurs de rNN50is (in situ). D'une manière ou d'une autre, un prélèvement indiciel est effectué, soit (i) à l'automne dès l'enfouissement des RCS et incubé de manière à obtenir rNN50iv après un nombre de jpi empirique, soit (ii) en sortie d'hiver après un nombre de jpe préétabli ici de manière à obtenir rNN50is. Il n'est plus question d'échantillonner pondéralement la parcelle, mais simplement d'y effectuer un prélèvement indiciel.

*(i) Détermination de rNN50iv in vitro :* suivi de la cinétique rNN au sein d'un prélèvement de terre à l'aide de microsondes pendant n jpi

*Prélèvements indiciels de la ZAR, avantageusement à l'automne*

rNN50iv étant une mesure non pondérale, un simple *prélèvement (indiciel)* suffit. La ZAR prélevée est facilement identifiable à la présence de RCS tout juste enfouis. Il est aussi possible de jauger la profondeur de celle-ci sur la base du réglage de l'outil aratoire ayant effectué l'enfouissement. Lors de certains travaux culturaux simplifiés (TCS), l'enfouissement des pailles à l'aide de déchaumeurs à dent et à disques s'effectue sur 5 à 10 cm de profondeur. Le prélèvement à l'aide d'une truelle de cette zone, à divers endroits sur la parcelle si l'on veut, nous évite maintenant

d'avoir à sonder en profondeur le sol. Notons enfin et encore une fois que rNN50is et rNN étant des ratios sans dimensions, il n'est plus nécessaire d'obtenir la densité apparente de la ZAR afin d'exprimer Nm en kg-N/ha.

La ZAR automnale est donc facilement repérable en profondeur par la présence de RCS enfouis ; cette profondeur dépendra de l'outil aratoire et sont réglage. Une fois le prélèvement unique, mais représentatif, effectué, quelques centaines de grammes de cette terre (ZAR) sont placés dans un bac prévu à cet effet, avantageusement et par exemple selon EP13366004.3, muni de deux microsondes, l'une pour l'azote nitrique, l'autre pour l'azote ammoniacal.

Les prélèvements sont placés dans des bacs protégeant leurs intégrités microbiologiques. Je propose à cet effet le kit de prélèvement environnemental EP2684588 permettant d'apporter au prélèvement un substrat liquide. En effet, sans avoir à rompre le scellé protégeant le prélèvement immédiatement après avoir été transféré du sol au bac, nous incubons cette terre pendant une période déterminée. Ce suivi de rNN in vitro à travers le temps fait ici office d'échantillonnage dudit prélèvement.

*Stratégie de modélisation Stics™*

Pour démontrer la faisabilité de la réalisation de l'invention et d'un méta-modèle décrivant la cinétique de rNN pour prédire rNN50 (infra), j'ai fait appel au modèle agronomique Stics™ (Brisson et al. 2008, Houlès et al. 2004). Une trentaine de situations agronomiques (Tableau 1) avec est sans azotobactérisation des RCS et permettant de déterminer eAZB (Tableau 2).

Les valeurs de rNN50iv calculées via l'équation (1) - une vingtaine, sont mises en relation avec les eAZB correspondant (Figure 4). J'ai réglé TMN et TMX, températures minimales et maximales, sans recourir au générateur de données quotidienne MODAWEC (cf. infra rNN50is), à 19 et 21 degrés C sur 365 jours ; la PCP (précipitation) est fixée à 2 mm par jours, soit un cumul de 730 mm par année, valeur moyenne pour la France. Les valeurs pour l'ensoleillement et l'évapotranspiration sont fixes et sans importance puisqu'il n'y a pas de biomasse végétale in vitro.

*Suivi de la cinétique rNN :: jpi et détermination de rNN50iv*

On mesure directement rNN de la ZAR à l'aide de microsondes, l'une pour N-NO$_3$ (Bendikov et al. 2005, Larsen et al. 1996) et l'autre pour N-NH$_4$ (Yang et al. 2011) directement à travers la membrane microporeuse du kit de prélèvement, par exemple selon EP13366004.3. Ces teneurs en N-NO$_3$ et N-NH$_4$ seront notées (automatiquement) toute au cours de la période d'incubation, aussi souvent que nécessaire pour tracer des courbes rNN :: jpi, sigmoïdes pour la plupart (Figure 3). Certains développements expérimentaux sont à prévoir afin de réduire les coûts de tels suivis, d'où à ce stade l'intérêt de la modélisation.

**Tableau 1** : Sommaire statistiques des données pédoclimatiques des trente (30) situations agronomiques récoltés de 2011 à 2015 avec et sans azotobactérisation des RCS impliquées dans le calcul d'eAZB. (rCNsol - ratio carbone sur azote de la matière organique du sol ; N et Corg - azote et carbone du sol ; RAT - résidus automnaux d'azote minéral ; RSH - résidus sortie hiver d'azote minéral, ammoniacal (NH4) et nitrique (NO3) ; rNN - ratio N-NH4 sur N-NO3 d'un échantillon ou prélèvement de terre ; dpH = pHKCl - pHeau ; N et Prcs - teneurs en N et P des RCS ; rCN, CP et NPrcs - ratios C sur N, C sur P et N sur P des RCS.

|  | RCS BTH (blé n=14) | écart-type | RCS CLZ (colza n=6) | écart-type | RCS TSL (tournesol n=3) | écart-type | RCS ZEA (maïs n=7) | écart-type |
|---|---|---|---|---|---|---|---|---|
| rCNsol | 9 | 1 | 10 | 0 | 10 | 0 | 10 | 0 |
| Norg | 0,13 | 0,025 | 0,176 | 0,058 | 0,175 | 0,108 | 0,142 | 0,025 |
| Corg | 1,19 | 0,33 | 1,76 | 0,58 | 1,75 | 1,08 | 1,42 | 0,25 |
| Argile | 30,6 | 9,3 | 23 | 7 | 30 | 26 | 19,7 | 8,2 |
| RAT | 32,1 | 9,1 | 29,2 | 12,1 | 17 | 3,5 | 27,5 | 12,3 |
| RSH-NH4 | 2,93 | 2,55 | 5,5 | 1,26 | 3,93 | 1,26 | 3,45 | 3,41 |
| RSH-NO3 | 25,1 | 16,1 | 44,5 | 19,2 | 32,6 | 9,6 | 27 | 20,3 |
| RSH | 28,1 | 18,2 | 50 | 20,5 | 36,6 | 8,4 | 30,5 | 22,5 |
| rNN | 0.108 | 0.06 | 0,117 | 0,022 | 0,118 | 0,071 | 0,135 | 0,065 |
| pHeau | 7,7 | 1,2 | 6,7 | 0,2 | 6,7 | 0,2 | 7,3 | 0,9 |
| pHKCl | 6,76 | 1,31 | 5,66 | 0,5 | 5,66 | 0,41 | 6,45 | 0,83 |
| dpH | 0,91 | 0,33 | 1,04 | 0,3 | 1,08 | 0,47 | 0,87 | 0,21 |

(suite)

| | RCS BTH (blé n=14) | écart-type | RCS CLZ (colza n=6) | écart-type | RCS TSL (tournesol n=3) | écart-type | RCS ZEA (maïs n=7) | écart-type |
|---|---|---|---|---|---|---|---|---|
| **RCS** | 9 | 0 | 6 | 0 | 6 | 0 | 12 | 0 |
| **Nrcs** | 2,14 | 0 | 1,02 | 0 | 1,02 | 0 | 1,4 | 0 |
| **Prcs** | 0,065 | 0,017 | 0,07 | 0 | 0,07 | 0 | 0,125 | 0 |
| **rCNrcs** | 90 | 0 | 45 | 0 | 45 | 0 | 60 | 0 |
| **rCPrcs** | 3095 | 447 | 657 | 0 | 657 | 0 | 670 | 0 |
| **rNPrcs** | 34,4 | 5 | 14,6 | 0 | 14,6 | 0 | 11,2 | 0 |

**Tableau 2** : Jeux de données provenant des modélisations des cinétiques rNN50iv, in vitro, mis en relation avec eAZB, l'efficacité de l'azotobactérisation des RCS (cf. Figure 4).

| Situation | dX | dN | RAT | Jpe | rMM50iv | eAZB:: RUN'rdt | eAZB:: RDT | eAZB:: RUN'rdn | eAZ8:: RDN |
|---|---|---|---|---|---|---|---|---|---|
| 1 | 174 | 208 | 14 | 118 | 0,433 | 156 | 143,3 | 115 | 105,5 |
| 2 | 154 | 182 | 14 | 118 | 0,433 | 150 | 150,2 | 123 | 122,8 |
| 3 | 210 | 192 | 34 | 128 | 0,278 | 106 | 106,4 | 100 | 99,7 |
| 4 | 190 | 151 | 34 | 128 | 0,278 | 122 | 114,2 | 122 | 113,6 |
| 5 | 102 | 151 | 36 | 153 | 0,218 | 120 | 105,6 | 118 | 104,2 |
| 6 | 130 | 121 | 13 | | | 112 | 102,7 | 129 | 117,8 |
| 7 | 143 | 134 | 25 | 145 | 0,176 | 120 | 108,5 | 105 | 95,2 |
| 8 | 133 | 139 | 25 | 145 | 0,176 | 106 | 101 | 101 | 96,3 |
| 9 | 56 | 99 | 10 | 143 | 0,289 | 133 | 107,8 | 128 | 104,1 |
| 10 | 152 | 127 | 24 | 10 | 0,429 | 133 | 122,3 | 139 | 127,9 |
| 11 | 173 | 136 | 53 | 158 | 0,182 | 98 | 90,7 | 105 | 97,8 |
| 12 | 155 | 132 | 8 | 153 | 0,368 | 118 | 108,5 | | |
| 13 | 205 | 110 | 21 | 148 | 0,223 | 114 | 110,7 | 117 | 113 |
| 14 | 180 | 115 | 19 | 150 | 0,248 | | 100 | 112 | 100 |
| 15 | 160 | 104 | 19 | 150 | 0.203 | | 104 | 116 | 111 |
| 16 | 140 | 91 | 19 | 150 | 0,203 | | 104 | 115 | 115 |
| 17 | 180 | 252 | 21 | 171 | 0,227 | | 104 | 110 | 99 |
| 18 | 160 | 247 | 21 | 171 | 0,227 | | 100 | 102 | 100 |
| 19 | 140 | 219 | 21 | 171 | 0,227 | | 107 | 108 | 104 |
| 20 | 210 | 119 | 23 | 140 | 0,28 | | 100 | 108 | 100 |
| 21 | 190 | 104 | 23 | 140 | 0,28 | | 106 | 112 | 109 |
| 22 | 170 | 98 | 23 | 140 | 028 | | 121 | 126 | 121 |
| 23 | 177 | 349 | 19 | 16 | | 94,5 | 94,7 | 85 | 85,4 |
| 24 | 177 | 301 | 19 | 16 | | 113 | 101,6 | 103 | 92,4 |
| 25 | 162 | 277 | 32,4 | 17 | | 134 | 119 | 103 | 91,5 |
| 26 | 162 | 259 | 32,4 | 17 | | 115,9 | 114,2 | 92 | 92,1 |
| 27 | 179 | 311 | 39,7 | 32 | | 98,1 | 98,2 | 92 | 91 |
| 28 | 179 | 266 | 39,7 | 32 | | 108,5 | 96,1 | 105 | 92,9 |
| 29 | 198 | 230 | 43,8 | 18 | 0,165 | 107,3 | 97 | 103 | 94,6 |
| 30 | 198 | 238 | 43,8 | 18 | 0,165 | 110 | 108,5 | 106 | 107 |

rNN50iv est donc repéré après une une phase accélérée d'augmentation de rNN selon jpi (Figure 3), rNN50iv étant ici la valeur rNN atteignant 50% du maximum associé à une inflexion de ladite accélération selon une fonction logistique, à quatre paramètres par exemple, du type ;

$$rNN = a + (d\ -a) / [\ 1 + (jpi\ /\ c)^b\ ] \qquad \text{équation (1)}$$

le paramètre c correspondant à l'abscisse à ce point de mi-pente dont l'ordonnée est (a+b)/2. Comme nous le verrons, rNN50iv (in vitro) s'avère être un indice d'eAZB comparable à rNN50is dérivé d'rNN (in situ), ce qui sera d'un point de vue technique, très avantageux pour l'utilisateur. Les courbes obtenues par modélisation Stics (Brisson et al. 2000, Houlès et al. 2004) ayant servies à cet effet sont décrites à la Figure 3.

*Détermination de eAZB selon rNN50iv*

Une fois rNN50iv identifié à l'aide de l'équation (1) appliquée aux courbes de types décrits à la Figure 3, on note que ces valeurs de rNN50iv sont assez bien corrélées avec eAZB (Tableau 3, Figure 4). En effet, ici la vingtaine situation pédologiques (Tableau 1) traduites en une trentaine de modélisations Stics (Brisson et al. 2000, Houlès et al. 2004) ont chacune avec une valeur d'eAZB à l'appui (Tableau 3). Or, et comme nous le voyons à la Figure 4, les corrélations eAZB :: rNN50iv sont bel et bien plus précises que celles, eAZB :: RAT, à la Figure 3, ce qui représente ici un net avantage technique lors du calcul de la dX (infra, *Applications*).

***(ii) Détermination de rNN50is in situ :*** *calcul* d'rNN50is à partir d'un estimé de rNN50iv après *n* jpe (jours post enfouissement des RCS)

*Stratégie de modélisation Stics*

La modélisation des rNN50is :: jpe* est plus complexe que celle des rNN50iv parce que rNN50is est défini comme le ratio rsh/rat tenant compte de l'éventuelle lixiviation des RAT. Néanmoins, les 20 sites (essais ; Tableau 1) comportant els 30 valeurs d'eAZB (Tableaux 2 et 3) constituent en fait quatre paramètres RCS (TLS, BTH, CLZ et ZEA), 20 paramètres sols (1 à 20 ; Tableau 1), et six paramètres climats (ASPH et BLGN 0, 1 et 2 ; Tableau 4). Ces 20 situations agro - pédologiques (sols) seront dont appliquées aux quatre (4) itinéraires techniques comportant différents RCS, et cela pour chacun des six (6) climats ; il en résultera donc 4 x 6 x 20 = 480 scenarii (modélisations) déroulés sur 210 jpe, soit 120 par type de RCS.

Ces modélisations permirent d'identifier à quel moment (jpe*) la valeur de rNN du prélèvement est la plus révélatrice de rNN50is. Les blés semés dans la foulée de l'enfouissement ont donc produit peu de biomasse, soit en dessous des ~ 1,5 t/ha critiques suggérées par Juste et al. 1994 pour la détermination de NNI. Pour chacun des 4 types de RCS, les 6 météorologies (Tableau 4) et les 20 situations pédoclimatiques (Tableau 1) sont décrites ici ; nous parlerons donc des 480 modélisations agro-pédo-climatiques, ou APC.

Les scénarii météorologiques sont générés à partir des moyennes climatiques (Tableau 4) à l'aide du logiciel MODAWEC (Liu et al. 2009) développé pour générer des précipitations quotidiennes sur 365 jours, ainsi que les maxima et minima de températures sur la base de valeurs mensuelles moyennes y compris le nombre de jours pluvieux, mesure indirecte de l'intensité des pluies. Liu et al. 2009 ont démonté que les rendements des cultures et l'évapotranspiration ainsi simulées se comparent très bien avec celles simulées avec les données météorologiques quotidiennes observées. MODAWEC peut donc étendre l'application d'un modèle tel que Stics™ dans les régions où les données quotidiennes ne sont pas disponibles, ou permettre des modélisations géographiquement plus fines.

**Tableau 3** : Exemple de données climatologiques de deux des six de données (ASPH 0 et BLGN 0) avec moyennes mensuelles de WTDA (nombre de jours pluvieux), PCP (pluviométrie ; mm), SDPC (écart-type PCP), TMX (température maximale ; degrés C), SDTX (écart-type TMX), TMN (température minimale ; degrés C), SDTN (écart-type TMN). MODAWEC (Liu et al. 2009) génère par la suite des valeurs météorologiques utilisables avec Stics™.

| ASPH 0 | JAN | FEB | MAR | APR | MAY | JuN | JUL | AUG | SEP | OCT | NOV | DEC |
|---|---|---|---|---|---|---|---|---|---|---|---|---|
| WTDA | 12 | 11,67 | 12,67 | 10,83 | 15,33 | 10,83 | 11,17 | 12,5 | 11,17 | 15,83 | 10 | 15 |
| PCP | 77,25 | 63,17 | 60,08 | 54,25 | 66,47 | 75,25 | 80,25 | 80,45 | 63,5 | 78,58 | 95,42 | 162,25 |
| SDPC | 8,04 | 7,28 | 9,33 | 7,89 | 5,54 | 8,11 | 8,83 | 11,24 | 8,97 | 6,17 | 13,83 | 17,07 |
| TMX | 3,1 | 3,94 | 9,92 | 15,49 | 19,59 | 22,87 | 25,33 | 25.41 | 20,35 | 15,06 | 9,01 | 4.6 |
| SDTX | 6,01 | 5,38 | 5,91 | 5,2 | 5,87 | 4,31 | 4,53 | 4,45 | 4,51 | 5,04 | 4,96 | 5,81 |
| TMN | -1,7 | -2,2 | 1,43 | 5,22 | 9,3 | 12,46 | 14,32 | 13,86 | 10,15 | 6,61 | 3,08 | -0,4 |
| SDTN | 5.43 | 5.44 | 5,67 | 4,26 | 4,17 | 3,44 | 3.21 | 3,16 | 3,98 | 4,57 | 4,18 | 5,06 |

| BLGN 0 | JAN | FEB | MAR | APR | MAY | JuN | JUL | AUG | SEP | OCT | NOV | DEC |
|---|---|---|---|---|---|---|---|---|---|---|---|---|
| WTDA | 15,5 | 7 | 15,5 | 7,5 | 15,5 | 15 | 15,5 | 15,5 | 15 | 15,5 | 15 | 15,5 |
| PCP | 64,5 | 3 | 34,5 | 2,5 | 36,5 | 5,5 | 33,5 | 2 | 96,5 | 6,5 | 51,5 | 4,5 |
| SDPC | 3,93 | 0,65 | 2,46 | 0,36 | 2,69 | 0,56 | 1,98 | 0,13 | 7,38 | 0,53 | 3,45 | 0,59 |
| TMX | 8,75 | 11,7 | 14,65 | 20,7 | 22,7 | 25,4 | 28,25 | 29,7 | 26,85 | 20,65 | 15,05 | 11,1 |
| SDTX | 197,39 | 166,81 | 223,17 | 211,24 | 275,88 | 278,72 | 284,86 | 336,27 | 326,74 | 278,27 | 200,82 | 217,64 |
| TMN | 0,9 | 2,35 | 5,8 | 8,85 | 11,75 | 14,35 | 16,95 | 16,35 | 13,9 | 9,6 | 7,1 | 2,45 |
| SDTN | 192,87 | 161,92 | 218,45 | 208,95 | 270,21 | 271,53 | 281,43 | 333,46 | 321,75 | 274,22 | 195,52 | 210,94 |

EP 3 479 671 B1

**Tableau 4** : Données pour les itinéraires technique selon le type de RCS. Ce sont ces valeurs qui ont été inscrites dans les fichers Stics .ini et .tec. Elles correspondent approximativement à des enfouissements des RCS à mi-août pour les RCS de blé tendre ou céréales (BTH) et colza (CLZ), à la mi-octobre pour les RCS de tournesol (TSL), et début novembre pour ceux de maïs-grain (ZEA). Les moments propices pour les prélèvements en jpe (jours post-enfouissement) ont été déterminés empiriquement selon le degré de corrélation rNN50is :: rNN$_{jpe*}$ (Figure 5).

| type de RCS | enfouissement (jj) | prélèvement (jj) | prélèvement (jpe*) | t/ha | rCN |
|---|---|---|---|---|---|
| TSL | 287 | ~ mi janvier | 60 | 7 | 45 |
| BTH | 228 | - mi janvier | 150 | 9 | 60 |
| CLZ | 228 | ~ mi janvier | 150 | 6 | 45 |
| ZEA | 312 | ~ mi janvier | 60 | 12 | 90 |

*Détermination de jpe\* et obtention de rNN50is selon le type de RCS*

La valeur de jpe* à laquelle déterminer rNN in situ (rNN50is) est établie empiriquement, soit par suivi in situ des rNN sur 30 cm de profondeur (développement expérimental assez onéreux), soit plus avantageusement ici par modélisation. jpe* est donc le jpe parmi les susdits 210 jpe auquel la relation rNN50is :: rNN est la plus forte. Il fallut ici donc observer, pas par pas, chacune desdites relations pour chaque jpe et s'arrêter sur jpe* pour lequel la corrélation rNN50is :: rNN est la plus forte. Selon cette étude, les jpe* pour les RCS TSL et ZEA sont d'environs 60, et d'environs 150 pour les RCS BTH et CLZ (Tableau 5). Or, ces relations rNN50is :: rNN$_{jpe}$ (Figure 5) sont foncièrement logistiques (sigmoïdales), et cela avantageusement à quatre paramètres du type ;

$$\text{rNN50is} = a + (d - a) / [\ 1 + (\text{rNNjpe*} / c)^b\ ] \qquad \text{équation (2).}$$

Les paramètres a et d sont des paramètres d'asymptotes (a étant le minimum et d le maximum), et b est le paramètre de pente. Le paramètre c correspond à l'abscisse du point de mi-pente dont l'ordonnée est (a+b)/2. Lorsque a est inférieur à d, la courbe descend de d à a et, lorsque a est supérieur à d, la courbe monte de a à d. Ces paramètres, pour chaque type de RCS, tel qu'estimés à partir des graphes rNN50is :: rNN (Figure 5) sont rapportés au Tableau 6. Plutôt imprécises à ce stade, ces relations feront maintenant l'objet de raffinements.

**Tableau 5** : Paramétrage de l'équation (2). Ces paramètres sont propres aux courbes logistiques avec asymptotes présentées à la Figure 5. Ces relations empiriques permettent ainsi de transformer les rNN in situ en rNN50is, variable in situ non pondérale ici particulièrement bien corrélée l'efficacité de l'azotobactérisation des RCS (eAZB ; Tableaux 5 et 6). Ces paramètres ne sont pas définitifs et pourront être peaufinés dans le cadre de développements expérimentaux.

| Paramètre | TSL | BTH | CLZ | ZEA |
|---|---|---|---|---|
| a | 17,00 | 3333 | 4,000 | 16,00 |
| b | 6,500 | 1,275 | 25,00 | 10,00 |
| c | 0,225 | 58,00 | 0,125 | 0,240 |
| d | 1,750 | 0,500 | 1,750 | 2,000 |

*Prélèvements indiciels de la ZAR en sortie hiver et scénario de modélisation*

Au lieu d'incuber in vitro le prélèvement de terre et suivre rNN à travers le temps (jpi) comme c'est le cas avec rNN50iv, on prélève la terre à un moment donné, jpe*, i.e. *n* jpe (jours post enfouissement), nécessairement avant le premier apport d'azote après la sortie d'hiver. Cela présuppose donc d'avoir préétablit jpe*, et cela pour chaque type de RCS (Tableaux 4 et 5) ; l'utilisateur n'aura qu'à prélever la ZAR, essentiellement de la même manière que pour la détermination de rNN50iv (supra), dans la mesure où la ZAR est toujours discernable en profondeur ; faut de quoi, un prélèvement à 30 cm de profondeur est préconisé. Or comme nous le verrons au Tableau 5, jpe*, moment auquel établir les rNN servant à établir rNN50is (Tableau 6, Figure 5) sont de 60 pour les RCS de TSL et ZEA, et de 150 pour ceux de BTH et CLZ. Notons encore une fois que rNN50is est une mesure non-pondérale de la capacité du sol à fournir de l'Nm pendant l'interculture, y compris ici l'Nm d'origine azotobactérienne via le traitement AZB des RCS.

*Établissement de la fonction rNN50is :: rNNjpe*

Le dosage des N-NO$_3$ et N-NH$_4$ des prélèvements in situ à n jpe est par contre cette fois-ci effectué conventionnellement par un laboratoire d'analyse. Ce faisant, ce prélèvement sera ainsi nécessairement échantillonné.

Il est aussi plausible de décrire directement rNN50is selon les rNN à jpe*, moment auquel les relations rNN50is :: rNN sont les plus significatives (Figure 5). Nb. rNN50is est le ratio RSH / RAT de façon à tenir compte de la lixiviation éventuelle d'Nm pendant l'interculture. Pour chaque type de RCS, les rNN50is et rNN jusqu'à 210 jpe pour chacune des 20 x 6 = 120 modélisations sont donc mises en relations. Nous obtenons ainsi des jeux de 40 à 120 points rNN50is :: rNN selon les modélisations retenues (Figure 5). Or, les jpe auxquels les relations rNN50is :: rNN sont les plus fortes (jpe*) dépend du type de RCS ; 60 dans le cas des RCS TSL et ZEA, et 150 pour les RCS de type BTH et CLZ (Tableau 4), et de la date d'enfouissement des RCS.

*Détermination de eAZB selon rNN50is*

Connaissant rNN du prélèvement effectué à jpe* (rNN$_{jpe*}$), nous pouvons maintenant calculer directement rNN50is pour cette parcelle, et du coup déterminer eAZB (Tableaux 6 et 7). Pour ce faire, rappelons que parmi les 4 x 6 x 20 = 480 scenarii modélisés ayant perms la formation des courbes rNN50is :: rNN (Figure 5) et rNN50iv :: jpi (Figure 3), trente (30) sont associés à de véritables valeurs d'eAZB (Tableau 3). Nous pouvons donc modéliser rNN à jpe* et déterminer à l'aide de l'équation (2) et des paramètres au Tableau 6 rNN50is pour chacun des trente (30) essais agronomiques selon leur type de RCS (Tableau 7).

**Tableau 6** (cf. Figure 6) : Efficacité de l'azotobactérisation des RCS (eAZB ; témoin = 100) pour 30 situations agronomiques comprenant quatre (4) types de résidus de culture au sol (RCS) ; tournesol (TSL), blé tendre d'hiver (BTH), colza (CLZ) et maïs-grain (ZEA). Les rendements agronomiques (RDT ; qx/ha) et protéique (RDN ; kg-N/ha) de blé tendre d'hiver (BTH), ainsi que les rendements unitaires - agronomiques ou protéiques (RUN ; RDT ou RDN par uN$_{fertilisant}$).

| Situation | RAT | rN$_{Njpe*}$ | rNN50is logit | eAZB:: RUNrdt | eAZB::RDT | eAZB:: RUNrdn | eAZB:: RDN |
|---|---|---|---|---|---|---|---|
| 1 | 14 | 0,143 | 3,927 | 156 | 143,3 | 115 | 105,5 |
| 2 | 14 | 0,143 | 3,927 | 150 | 150,2 | 123 | 122,8 |
| 3 | 34 | 0,114 | 1,959 | 106 | 106,4 | 100 | 99,7 |
| 4 | 34 | 0,114 | 1,959 | 122 | 114,2 | 122 | 113,6 |
| 5 | 36 | 0,146 | 2,116 | 120 | 105,6 | 118 | 104,2 |
| 6 | 13 | 0,200 | 6,591 | 112 | 102,7 | 129 | 117,8 |
| 7 | 25 | 0,153 | 2,152 | 120 | 108,5 | 105 | 95,2 |
| 8 | 25 | 0,153 | 2,152 | 106 | 101 | 101 | 96,3 |
| 9 | 10 | 0,221 | 6,307 | 133 | 107,8 | 128 | 104,1 |
| 10 | 24 | 0,100 | 2,002 | 133 | 122,3 | 139 | 127,9 |
| 11 | 53 | 0,129 | 1,874 | 98 | 90,7 | 105 | 97,8 |
| 12 | 8 | 0,260 | 3,865 | 118 | 108,5 | | |
| 13 | 21 | 0,200 | 3,947 | 114 | 110,7 | 117 | 113 |
| 14 | 19 | 0,045 | 0,865 | | 100 | 112 | 100 |
| 15 | 19 | 0,045 | 0,865 | | 104 | 116 | 111 |
| 16 | 19 | 0,045 | 0,865 | | 104 | 115 | 115 |
| 17 | 21 | 0,064 | 1,063 | | 104 | 110 | 99 |
| 18 | 21 | 0,064 | 1,063 | | 100 | 102 | 100 |
| 19 | 21 | 0,064 | 1,063 | | 107 | 108 | 104 |
| 20 | 23 | 0,147 | 2,130 | | 100 | 108 | 100 |
| 21 | 23 | 0,147 | 2,130 | | 106 | 112 | 109 |
| 22 | 23 | 0,147 | 2,130 | | 121 | 126 | 121 |
| 23 | 19 | 0,077 | 1,765 | 94,5 | 94,7 | 85 | 85,4 |
| 24 | 19 | 0,077 | 1,765 | 113 | 101,6 | 103 | 92,4 |
| 25 | 32,4 | 0,101 | 1,509 | 134 | 119 | 103 | 91,5 |
| 26 | 32,4 | 0,101 | 1,509 | 115,9 | 114,2 | 92 | 92,1 |

(suite)

| Situation | RAT | rN_Njpe* | rNN50is logit | eAZB:: RUNrdt | eAZB::RDT | eAZB:: RUNrdn | eAZB:: RDN |
|---|---|---|---|---|---|---|---|
| 27 | 39,7 | 0,094 | 1,752 | 98,1 | 98,2 | 92 | 91 |
| 28 | 39,7 | 0,094 | 1,752 | 108,5 | 96,1 | 105 | 92,9 |
| 29 | 43,8 | 0,058 | 2,000 | 107,3 | 97 | 103 | 94,6 |
| 30 | 43,8 | 0,058 | 2,000 | 110 | 108,5 | 106 | 107 |

L'efficacité de i'azotobactérisation des RCS (eAZB) est relative à un témoin non traité (TNT = 100) et peut donc être exprimée en termes de RDT, RDN et/ou RUN', au choix. Afin de permettre le lissage des valeurs, deux par deux, les trente observation (autant d'essais) ont été ordonnée par eAZB :: RDN grandissante. J'ai constaté net un progrès technique par rapport aux mesures concernant les teneurs en Nm du sol les plus immédiatement comparables à rNN50is, et cela à l'exclusion des RSH eux-mêmes, mesures trop pondérales et donc imprécises. De plus, cette corrélation eAZB :: rNN50is est plus forte que la corrélation eAZB :: rNN (en abîme, Figure 6), ou encore plus simplement eAZB :: RAT (EP2942621), et cela à hauteur de 10 à 20% en termes de $R^2$. Cette plus grande précision sera valorisée au moment de l'intégration (l'agrégation) d'rNN50is dans un indice composé encore plus révélateur de pAZB et eAZB.

Tableau 7 : *Idem* au Tableau 6 - Les moyennes glissantes (*moving average* ; n= 3) de manière à *graduer* la réponse d'eAZB à rNN50is. Les valeurs au Tableau 7 ayant été ordonnées selon RDN (rendement protéique ; kg protéique par unité d'azote fertilisant apporté), la réponse d'eAZB :: RUN'rdn à la Figure 6 est d'autant plus non biaisée. Le type de météorologie (Tableau 4) et les identifiant USM permettent de retrouver ces 30 essai parmi les 480 scenarii ayant servis à la construction des courbes rNN50is :: rNN_jpe* à la Figure 5.

| Situation | RAT | rNN_jpe* | rNN50is liné | rNN50is logit | eAZB:: RUN'rdt | eAZB:: RDT | eAZB:: RUN'rdn | eAZB:: RDN |
|---|---|---|---|---|---|---|---|---|
| 1 | 28,9 | 0,100 | 1,820 | 2,964 | 133 | 126 | 111 | 106 |
| 2 | 19,0 | 0,122 | 2,539 | 2,965 | 142 | 136 | 131 | 125 |
| 3 | 26,5 | 0,080 | 1,535 | 1,412 | 106 | 103 | 106 | 100 |
| 4 | 26,5 | 0,080 | 1,535 | 1,412 | 122 | 109 | 119 | 114 |
| 5 | 25,0 | 0,145 | 2,543 | 3,021 | 138 | 124 | 117 | 105 |
| 6 | 18,0 | 0,173 | 3,729 | 4,360 | 112 | 112 | 128 | 119 |
| 7 | 25,0 | 0,153 | 3,814 | 2,152 | 113 | 105 | 103 | 96 |
| 8 | 39,0 | 0,141 | 2,812 | 2,013 | 102 | 96 | 103 | 97 |
| 9 | 23,0 | 0,184 | 4,083 | 4,211 | 127 | 107 | 123 | 104 |
| 10 | 16,0 | 0,180 | 2,761 | 2,934 | 126 | 115 |  |  |
| 11 | 37,0 | 0,096 | 1,393 | 1,469 | 98 | 97 | 108 | 98 |
| 12 |  |  |  |  |  |  |  |  |
| 13 | 27,5 | 0,157 | 3,871 | 2,953 | 118 | 112 | 120 | 113 |
| 14 | 20,0 | 0,055 | 0,857 | 0,964 |  | 100 | 107 | 100 |
| 15 | 20,0 | 0,123 | 3,075 | 2,406 | 114 | 107 | 117 | 112 |
| 16 | 16,0 | 0,123 | 3,075 | 3,728 | 112 | 103 | 122 | 116 |
| 17 | 27,5 | 0,089 | 1,653 | 1,511 | 106 | 105 | 105 | 99 |
| 18 | 22,0 | 0,105 | 1,511 | 1,596 |  | 100 | 105 | 100 |
| 19 | 15,5 | 0,143 | 3,555 | 3,685 | 133 | 107 | 118 | 104 |
| 20 | 22,0 | 0,105 | 1,511 | 1,596 |  | 104 | 108 | 102 |
| 21 | 21,0 | 0,096 | 1,393 | 1,497 |  | 105 | 114 | 110 |
| 22 | 18,5 | 0,145 | 2,549 | 3,029 | 150 | 136 | 125 | 122 |
| 23 |  |  |  |  |  |  |  |  |
| 24 | 29,4 | 0,086 | 1,970 | 1,758 | 111 | 99 | 104 | 93 |
| 25 | 32,4 | 0,101 | 1,453 | 1,509 | 125 | 117 | 98 | 92 |
| 26 | 25,7 | 0,089 | 1,779 | 1,637 | 114 | 108 | 98 | 92 |
| 27 | 36,1 | 0,097 | 1,643 | 1,630 | 116 | 109 | 98 | 91 |
| 28 | 41,8 | 0,076 | 1,211 | 1,876 | 108 | 97 | 104 | 94 |

(suite)

| Situation | RAT | rNN$_{jpe*}$ | rNN50is liné | rNN50is logit | eAZB:: RUN'rdt | eAZB:: RDT | eAZB:: RUN'rdn | eAZB:: RDN |
|---|---|---|---|---|---|---|---|---|
| 29 | 34,4 | 0,105 | 2,200 | 2,076 | 114 | 103 | 104 | 95 |
| 30 | 33,4 | 0,102 | 1,317 | 2,065 | 110 | 107 | 109 | 108 |

## APPLICATIONS BIOINDUSTRIELLES ET AGRONOMIQUES

rNN50iv et rNN50is sont nettement plus déterminants d'eAZB que les simples RAT et RSH. Cela permet d'appliquer ces indices à la FAR (fertilisation azotobactérienne raisonnée ; EP17196251.7). De plus, ces indices sont avant-gardistes en ce sens qu'ils sont issus de *pi* plutôt que d'*ep*.

rNN50iv et rNN50is seront intégrés, agrégés en sorte, dans un indice composé. Nous prévoyons qu'il sera ainsi possible non seulement de prédire eAZB, mais aussi et surtout, de recalculer les besoins en engrais N de la culture en termes de dX, mais tenant maintenant compte maintenant de telles azotobactérisations des RCS, fait nouveau pour le secteur de la fertilisation-N raisonnée des grandes cultures. En effet, rNN50iv et rNN50iv sont assez bien corrélés à eAZB. Il est donc possible d'utiliser ces indices comme prédicteurs d'eAZB affectant la culture suivante implantée sur ces différents types de RCS et la ZAR ainsi constituée. Les azotobactéries inoculées dans la ZAR vont nécessairement affecter sa contribution en Nm. Mais ce qu'il y a de plus intéressant ici c'est que les indices rNN50iv et/ou rNN50is sont assez bien corrélés avec eAZB ; nous pouvons donc prédire eAZB à l'aide de ces indices, et du coup mieux rationner, raisonner en sorte, la fertilisation N en présence de telles biofertilisations. Bien qu'imparfaites, ces relations eAZB :: rNN50iv (Figure 4) et eAZB :: rNN50is sont suffisamment fortes, même à ce stade de développement de la technologie, pour servir d'indice d'eAZB. In fine, l'invention contribue à la *fertilisation azotobactérienne raisonnée* (FAR ; Figure 8 ; Claude et Fillion 2004 ; Claude 2017 ; EP17196251.7) en proposant un nouvel indicateur élémentaire d'eAZB, iCAS, pour le calcul de la dose prévisionnelle d'engrais-N, dite « dN » plutôt que « dX » pour marquer la différence avec la méthode de bilan. La FAR s'appuie sur la modélisation dynamique des cinétiques C, N, P et S en sols arables, et donc sur l'exploitation des bases de données agro-écologiques françaises, européennes, voire mondiales (Dumanski et al. 1993).

### Références, bibliographie et brevets pertinents

Abras, J-P Goffart et J-P Destain. 2013. Perspectives d'amélioration du conseil prévisionnel de fertilisation azotée à la parcelle en Wallonie par l'utilisation du logiciel AzoFert®. Biotechnol. Agron. Soc. Environ. 2013 17(S1), 215-220

Bendikov, TA., J Kim et TC. Harmon. 2005. Development and environmental application of a nitrate selective microsensor based on doped polypyrrale films. Sensors and Actuators B 106:512-517

Brisson, M. Launay, B. Mary et N. Beaudoin (eds) 2008. Conceptual basis, formalisations and parameterization of the STICS crop model. Éds. Quae (Inra), RD10 78026 versailles Cedex, France.

Cambardella CA, TB Moorman, JM Novak, TB Parkin, DL Karlen, RF Turco et AE Konopka. 1985. Field scale variability of soil properties in central Iowa soils. Soil Sci. Soc. Am. J. 58:1501-1511.

Claude, P.-P., MacKenzie, A. F., Coulman, B. E. et Mehuys, G. R. 1993. Effet du labour printanier et des cultures intercalaires de trbile rouge sur le rendement du maïs-grain. Can. J. Plant Sci. 73: 37-46. (http://www.nrcresearchpress.com/doi/pdfplus/10.4141/cjps93-006)

Claude, P-P. et M. Giroux. 2006. Effet des engrais organo-minéraux inoculés (EOMI) sur la croissance des plants de maïs-grain, les rendements, les prélèvements des éléments nutritifs et la qualité des grains. Agrosol. 17: 51-64. (https://www.irda.qc.ca/assets/documents/Publications/documents/claude-giroux-2006 article effet eomi mais.pdf)

Claude, P-P. et L. Fillion. 2004. Effet de l'apport d'un inoculum bactérien aux résidus de culture de maïs-grain au sol sur le rendement et la qualité de blés d'hiver panifiables en France. Agrosol 15: 23-29. (http://collections.banq.qc.ca/ark:/52327/bs604022)

Comifer 2013. Calcul de la fertilisation azotée : Guide méthodologique pour l'établissement des prescriptions locales. Comifer Le Diamant A, 92909 Paris La Défense Cedex Tél. : 01 46 53 10 75 Édition 2013

Constantin, J., N. Beaudoin, M. Launay, J. Duval et B. Mary. 2012. Long-term nitrogen dynamics in various catch crop scénarios: Test and simulations with STICS model in a temperate climate. Agriculture, Ecosystems and Environment 147 (2012) 36- 46

Dumanski, J., W.W. Pettapiece, D.F. Acton et P-P. Claude. 1993. Application of agro-ecological concepts and hierarchy theory in the design of databases for spatial and temporal characterization of land and soil. Geoderma 60 : 343-358. (http://www.sciencedirect.com/science/article/pii/001670619390035J)

Grignani, C et L Zavattaro. 2000. A survey on actual agricultural practices and their effects on the mineral nitrogen concentration of the soil solution. Eur. J. Agron. 12 : 251-268

Houlès, Vianney, Bruno Mary, Martine Guérif, David Makowski et Eric Justes. 2004. Evaluation of the ability of the crop model STICS to recommend nitrogen fertilisation rates according to agro-environmental criteria. Agronomie 24 (2004) 339-349 339

Justes, E., B. Mary et B. Nicolardot. 2009. Quantifying and modelling C and N mineralization kinetics of catch crop residues in soil: parameterization of the residue décomposition module of STICS model for mature and non-mature residues. Plant Soil 325 :171-185

Ladha, J. K. et al. 2016. Global nitrogen budgets in cereals: A 50-year assessment for maize, rice, and wheat production systems. Sci. Rep. 6, 19355; doi: 10.1038/srep19355 (2016)

Lairez, J, P Feschet, J Aubin, Ch Boskstaller et I Bouvarel. 2015. Agriculture et développement durable : guide pour l'évaluation multicritère. Eds. Quae, 78026 Versailles Cedex

Larsen, Lars Hauer, Niels Peter Revsbech et Svend Jørgen Binnerup. 1996. A Microsensor for Nitrate Based on Immobilized Denitrifying Bacteria. Appl. Environ. Microbiol. 62 :1248-1251

Liska, AJ, H Yang, M Milner, S Goddard, H Blanco-Canqui, MP Pelton, XX Fang, H Zhu et AE Suyker. 2014. Biofuels from crop residue can reduce soil carbon and increase CO2 émissions. Nature Climate Change | vol 4 | may 2014 | DOI: 10.1038/NCLIMATE2187

Liu, J, JR. Williams, X Wang et H Yang, 2009. Using MODAWEC to generate daily weather data for the EPIC model. Environmental Modelling & Software 24 (2009) 655-664

Manzoni, S et A Porporato. 2009. Soil carbon and nitrogen mineralization: Theory and models across scales. Soil Biology & Biochemistry 41 : 1355-1379

Mengel, K. 1990. Minéralisation de l'azote organique et optimisation de l'apport d'engrais azoté. C.R. Acad. Agric. France 76(8):3-12

Okajima, Hideo et Ichiro Taniyama (1980). Significance of mass flow in nitrate-nitrogen supply to plant roots. Soil Science and Plant Nutrition, 26:3, 363-374,

Recous, S., D. Robin, D. Darwis et B. Mary. 1995. Soil inorganic N availability: effect of maize residue décomposition. Soil Biol. Biochem. Vol. 27, No. 12, pp. 1529-1538, 1995

Richter, G.M. Richter, A.J. Beblik, K. Schmalstieg, et O. Richter. 1998. N-dynamics and nitrate leaching under rotational and continuons set-aside-a case study at the field and catchment scale. Agriculture, Ecosystems and Environment 68 1998. 125-138

Tabor, J.A., A.W. Warrick, D.E. Myers, and D.A. Pennington. 1985. Spatial variability of nitrate in irrigated cotton: II. Soil nitrate and correlated variables. Soil Sci. Soc. Am. J. 49:390-394.

Yang, Ming, Yunting Fang, Di Sun et Yuanliang Shi. 2016. Efficiency of two nitrification inhibitors (dicyandiamide and 3, 4-dimethypyrazole phosphate) on soil nitrogen transformations and plant productivity: a meta-analysis. Nature Scientific Reports | 6:22075 | DOI: 10.1038/srep22075

Yang, Ming-Zhi, Ching-Liang Dai and Chyan-Chyi Wu. 2011. A Zinc Oxide Nanorod Ammonia Microsensor Integrated with a Readout Circuit on-a-Chip. Sensors 11:11112-11121

**Revendications**

1. **Méthode pour la formation d'un indicateur élémentaire de l'efficacité de l'azotobactérisation (eAZB)** des résidus de culture cellulosiques au sol (RCS) et de culture intermédiaires pièges à nitrates (cipan) applicable en fertilisation raisonnée (FR) par mesure du ratio N-NH$_4$/N-NO$_3$ (rNN) de l'azote minéral (Nm) du sol,
   ledit rNN étant obtenu sans recourir à l'échantillonnage pondéral de la zone vadose ou d'enracinement et lorsque les RCS et/ou les cipans sont azotobactérisés par renforcement, **caractérisée en ce qu'**il compris via l'inoculation, des populations d'azotobactéries telluriques en proximité des RCS et des cipans avant leur enfouissement et la constitution de la ZAR, et **en ce que** ;

   • la *zone d'activité résidusphérique* (ZAR), c'est-à-dire le volume de sol entourant les susdits résidus de culture en cour de décomposition dans ou sur le sol et affectant l'activité des microflores microbiennes, fait l'objet d'un simple *prélèvement* indiciel échantillonné pondéralement pour Nm et rNN, et **en ce que** ;
   • la profondeur de la ZAR est égale à celle de l'enfouissement des RCS,
   • la valeur critique de rNN, rNN50, est déterminée soit in vitro (rNN50iv), soit in situ (rNN50is), et représente la valeur médiane entre les valeurs rNN minimales et maximales respectives atteintes au cour de la minéralisation desdits RCS à travers le temps.

**2.** Méthode pour formation d'un indicateur élémentaire de l'efficacité de l'azotobactérisation (eAZB) selon la première revendication **caractérisée en ce que** la ZAR est repérée visuellement par la présence manifeste de RCS à travers une coupe de la surface du sol, ou implicitement sur la base du réglage de l'instrument aratoire utilisé pour l'enfouissement des RCS tel que par exemple une charrue à socs ou encore des instruments aratoires à disques et dérivés de disques.

**3.** Méthode pour formation d'un indicateur élémentaire de l'efficacité de l'azotobactérisation (eAZB) selon une quelconque des précédentes revendications **caractérisée en ce que** la ZAR pour détermination de rNN50iv est <u>prélevée en automne</u> immédiatement après l'enfouissement des résidus de culture cellulosiques (RCS).

**4.** Méthode pour formation d'un indicateur élémentaire de l'efficacité de l'azotobactérisation (eAZB) selon une quelconque des revendications précédentes **caractérisée en ce que** le prélèvement de la ZAR est échantillonné de manière répétée afin de déterminer rNN à travers le temps et d'en établir la cinétique, avantageusement journalière, par exemple à l'aide de microsondes spécifiques pour l'N-NO$_3$ et N-NH$_4$.

**5.** Méthode pour formation d'un indicateur élémentaire de l'efficacité de l'azotobactérisation (eAZB) selon une quelconque des précédentes revendications précédentes **caractérisée en ce que** pour les RCS de types TSL (tournesol) et ZEA (maïs) enfouis tardivement à l'automne ou encore les RCS de types BTH (blé d'hiver, céréales) et CLZ (colza) enfouis hâtivement à la fin de l'été les prélèvements de la ZAR sont effectués entre 0 et 7 jpe (jours post enfouissement), et plus particulièrement dès que possible post enfouissement.

**6.** Méthode pour formation d'un indicateur élémentaire de l'efficacité de l'azotobactérisation (eAZB) selon une quelconque des précédentes revendications **caractérisée en ce que** le moment donné pour l'établissement de rNN50iv correspond à une augmentation accélérée de rNN au cours de l'incubation in vitro dudit prélèvement de la ZAR à température et humidité constantes, et plus particulièrement au moment correspondant au point d'inflexion d'une fonction empirique avec plateaux asymptotiques de type ;

$$rNN = a + (d -a) / [ 1 + (jpi / c)^b ]$$

sachant que les paramètres a et d sont des paramètres d'asymptotes (a étant le minimum et d le maximum), b est le paramètre de pente, le paramètre c correspond à l'abscisse du point de mi-pente, ou *point d'inflexion*, dont l'ordonnée est (a+b)/2 lorsque rNN = rNN50iv, et jpi (jours post initiation) la période de temps, en jours, écoulée entre le début et la fin de l'incubation in vitro du prélèvement de la ZAR.

**7.** Méthode pour formation d'un indicateur élémentaire de l'efficacité de l'azotobactérisation (eAZB) selon les revendications 1 et 2 **caractérisée en ce que** la ZAR pour détermination de rNN50is est <u>prélevée en sortie hiver</u>.

**8.** Méthode pour formation d'un indicateur élémentaire de l'efficacité de l'azotobactérisation (eAZB) selon la revendication précédente **caractérisée en ce que** le prélèvement de la ZAR est échantillonnée ponctuellement de manière répétée afin d'obtenir une mesure représentative et ponctuelle de rNN dans ce prélèvement.

**9.** Méthode pour formation d'un indicateur élémentaire de l'efficacité de l'azotobactérisation (eAZB) selon une quelconque des deux revendications précédentes **caractérisée en ce que** l'indice rNN50is est obtenu directement d'une fonction empirique décrivant rNN50is selon la valeur de rNN au moment des susdits prélèvements (jpe*), jpe* étant le jpe auquel la relation rNN50is :: rNN est la plus forte, et plus particulièrement à l'aide d'une fonction logistique avec asymptotes à quatre paramètres de type;

$$rNN50 = a + (d -a) / [ 1 + (rNN_{jpe*} / c)^b ] = rNN50is$$

sachant que les paramètres a et d sont des paramètres d'asymptotes (a étant le minimum et d le maximum), b est le paramètre de pente, et c le paramètre correspondant à l'abscisse du point de mi-pente dont l'ordonnée est (a+b)/2 lorsque rNN = rNN50is.

**10.** Méthode pour formation d'un indicateur élémentaire de l'efficacité de l'azotobactérisation (eAZB) selon une quelconque des trois revendications précédentes **caractérisée en ce que** le moment du prélèvement in situ de la ZAR, en nombre de jpe, le plus révélateur de rNN entant qu'indice de la contribution de Nm (jpe*) est situé, pour les RCS de types TSL et ZEA enfouis tardivement à l'automne, entre 50 et 70 jpe, et plus particulièrement à 60 jpe ; pour les RCS de types BTH et CLZ enfouis hâtivement à la fin de l'été, ce moment est situé entre 120 et 160 jpe, et plus particulièrement à environ 150 jpe.

11. Méthode pour formation d'un indicateur élémentaire de l'efficacité de l'azotobactérisation (eAZB) selon une quelconque des revendications précédentes **caractérisée en ce que** les valeurs rNN50iv et/ou rNN50is, étant elles corrélées à eAZB - i.e. efficacité de l'azotobactérisation du RCS en termes agronomiques (rendements en grains ou en protéines) par rapport à un témoin avec RCS non-azotobactérisés, sont utilisées entant qu'indice prédisant eAZB sur la culture suivante implantée sur ces RCS enfouis.

**Patentansprüche**

1. **Methode zur Bildung eines grundlegenden Indikators der Effizienz der Azotobakterisierung (eAZB)** von Resten von Cellulosepflanzen im Boden (RCS) und von Nitratbindendenden Zwischenkulturen (Cipan), die bei verantwortungsvoller Düngung (FR) anwendbar ist, durch die Messung des Verhältnisses $N-NH_4/N-NO_3$ (rNN) des mineralischen Stickstoffs (Nm) im Boden, dieses rNN wird ermittelt, ohne dabei auf Gewichts-Stichproben der ungesättigten Zone oder der Wurzelzone zurückzugreifen und wenn die RCS und/oder die Cipan durch Verstärkung azotobakterisiert werden, inklusive durch Beimpfung, Populationen von Boden-Azotobakterien in der Nähe der RCS und der Cipan, vor ihrem Einackern und der Konstituierung der ZAR,
und **dadurch gekennzeichnet, dass**;

   • die *Zone der residuenspezifischen Aktivität* (ZAR), also das Bodenvolumen, das die obengenannten Pflanzenreste umgibt, die in oder auf dem Boden zersetzt werden und die Aktivität der mikrobischen Mikroflora beeinflussen, Gegenstand einer einfachen *Index-Stichprobe* nach Gewicht für Nm und rNN ist, und dadurch dass;
   • die Tiefe der ZAR gleich jener der Ablagerung der RCS ist,
   • der kritische Wert des rNN, rNN50, wird entweder in vitro (rNN50iv) oder in situ (rNN50is) bestimmt und stellt den Medianwert zwischen dem jeweiligen Minimalwert und Maximalwert von rNN dar, der im Lauf der Mineralisierung der genannten RCS über die Zeit erreicht wird.

2. Methode zur Bildung eines grundlegenden Indikators der Effizienz der Azobakterisierung (eAZB) gemäß des ersten Patentanspruchs, dadurch charakterisiert, dass die ZAR optisch nach einem Schnitt der Bodenoberfläche anhand von manifester Präsenz von RCS zu erkennen ist oder sie Implizit auf der Basis der Einstellung des landwirtschaftlichen Geräts, das zum Eingraben der RCS, wie zum Beispiel ein Pflug mit Scharen oder Geräte mit Scheiben oder ähnlichem, zu finden ist.

3. Methode zur Bildung eines grundlegenden Indikators der Effizienz der Azobakterisierung (eAZB) gemäß eines der vorangehenden Patentansprüche, dadurch charakterisiert, dass die ZAR zur Bestimmung des rNN50iv im Herbst beprobt wird, direkt nach dem Eingraben der Reste der Cellulosepflanzen (RCS).

4. Methode zur Bildung eines grundlegenden Indikators der Effizienz der Azobakterisierung (eAZB) gemäß eines der vorangehenden Patentansprüche, dadurch charakterisiert, dass die Probe der ZAR wiederholt entnommen wird um die Veränderung des rNN im Verlauf der Zeit zu bestimmen und und um seine Kinetik zu bestimmen, am besten täglich, zum Beispiel mit der Hilfe von Mikrosonden, die spezifisch auf N-NO3 und N-NH4 reagieren.

5. Methode zur Bildung eines grundlegenden Indikators der Effizienz der Azobakterisierung (eAZB) gemäß eines der vorangehenden Patentansprüche, dadurch charakterisiert, dass für die RCS der Typen TSL (Sonnenblume) und ZEA (Mais), die spät im Herbst eingeackert werden, oder auch die RCS der Typen BTH (Winterweizen, Getreide) und CLZ (Raps), die hastig zu Ende des Sommers eingeackert werden, die Probenahme der ZAR zwischen 0 und 7 jpe (Tage nach dem Einackern), und genauer gesagt baldmöglichst nach dem Vergraben, erledigt wird.

6. Methode zur Bildung eines grundlegenden Indikators der Effizienz der Azobakterisierung (eAZB) gemäß eines der vorangehenden Patentansprüche, dadurch charakterisiert, dass der gegebene Moment für die Bestimmung des rNN50iv zusammenhängt mit einem beschleunigten Anstieg des rNN im Verlauf der in vitro-Inkubation der genannten Probe der ZAR bei konstanter Temperatur und Feuchtigkeit und insbesondere zum Moment der dem Wendepunkt einer empirischen Funktion mit asymptotischen Plateaus entspricht, der Art;

$$rNN = a + (d -a) / [ 1 + (jpi / c)b ]$$

wissend, dass die Parameter a und d Parameter der Asymptoten sind (a ist das Minimum und d das Maximum), b ist der Parameter der Steigung und der Parameter c entspricht der Abszisse am Punkt der mittleren Steigung, oder Wendepunkt, wo die Ordinate (a+b)/2 ist, wenn rNN = rNN50is und jpi (Tage nach Beginn) die Zeitperiode, in Tagen, verstrichen zwischen dem Beginn und dem Ende der in vitro-Inkubation der ZAR-Probe.

7. Methode zur Bildung eines grundlegenden Indikators der Effizienz der Azobakterisierung (eAZB) gemäß der Patentansprüche 1 und 2, dadurch charakterisiert, dass die ZAR zur Bestimmung des rNN50is am Ende des Winters beprobt wird.

8. Methode zur Bildung eines grundlegenden Indikators der Effizienz der Azobakterisierung (eAZB) gemäß des vorhergehenden Patentanspruches, dadurch charakterisiert, dass die Beprobung der ZAR punktuell und wiederholt durchgeführt wird, um eine repräsentative Messung des rNN bei dieser Probenahme zu erhalten.

9. Methode zur Bildung eines grundlegenden Indikators der Effizienz der Azobakterisierung (eAZB) gemäß eines jeglichen der beiden vorangegangenen Patentansprüche, dadurch charakterisiert, dass die Kennziffer rNN50is direkt aus einer empirischen Funktion erhalten wird, indem rNN50is nach dem Wert des rNN im Moment der genannten Probenahme (jpe*) beschrieben wird, jpe* ist dabei der jpe zu dem das Verhältnis rNN50is :: rNN am stärksten ist, und noch genauer gesagt unter Verwendung einer logistischen Funktion mit Asymptoten mit vier Parametern nach der Art;

$$rNN50 = a + (d -a) / [ 1 + (rNNjpe* / c)b ] = rNN50is$$

Wissend, dass die Parameter a und d Parameter der Asymptoten sind (a ist das Minimum und d das Maximum), b ist der Parameter der Steigung und c der Parameter, der der Abszisse am Punkt der mittleren Steigung entspricht, wo die Ordinate (a+b)/2 ist, wenn rNN = rNN50is.

10. Methode zur Bildung eines grundlegenden Indikators der Effizienz der Azobakterisierung (eAZB) gemäß eines jeglichen der drei vorangegangenen Patentansprüche, dadurch charakterisiert, dass der Moment der in situ-Probenahme der ZAR, in Anzahl der jpe, der am aussagekräftigsten über das rNN als Kennziffer für den Beitrag des Nm (jpe*) ist, liegt für RCS der Typen TSL und ZEA, die spät im Herbst eingeackert werden, zwischen 50 und 70 jpe, und insbesondere bei 60 jpe; für die RCS der Typen BTH und CLZ, die gleich mit Ende des Sommers eingeackert werden, liegt dieser Moment zwischen 120 und 160 jpe, genauer gesagt bei etwa 150 jpe.

11. Methode zur Bildung eines grundlegenden Indikators der Effizienz der Azobakterisierung (eAZB) gemäß einer der vorangegangenen Patentansprüche, dadurch charakterisiert, dass die Werte rNN50iv und/oder rNN50is mit dem eAZB korrellieren - d.h. die Effizienz der Azobakterisierung der RCS in agronomischer Hinsicht (Ertrag in Getreide oder Proteinen) im Vergleich mit einem Test mit nicht-azotobakterisierten-RCS, verwendet werden als Kennzahlen um den eAZB der Kultur vorherzusagen, die folgend auf die eingeackerten RCS gepflanzt wird.

**Claims**

1. **Method for the formation of an elementary indicator of the efficiency of azotobacterization (eAZB)** of cellulosic soil borne crop residues (RCS) and of nitrate catch crops (cipan) applicable in *integrated fertilizer mangement* (FR) by measurement of the N-NH4 / N-NO3 (rNN) ratio of mineral nitrogen (Nm) in the soil, the said rNN being obtained without resorting to sampling of the vadose or rooting zone and when the RCS and/or the cipans are azotobacterized by reinforcement **characterized in that** it comprises the inoculation of populations of telluric azotobacteria in the vicinity of the RCS and the cipans prior to their burial and the constitution of the ZAR,
and **in that** ;

   • the *residuspheric activity zone* (ZAR), i.e. the volume of soil surrounding the aforementioned crop residues in the process of decomposing in or on the soil and affecting the activity of microbial microflora, is the object of a simple indexed specimen obtained of Nm and rNN, and **in that**;
   • the depth of the ZAR is equal to that of the burial depth of the RCS,

• the critical value of rNN, rNN50, is determined either *in vitro* (rNN50iv) or *in situ* (rNN50is) and represents the median value between the respective minimum and maximum rNN values reached during the mineralization of said RCS over time.

2. Method for the formation of an elementary indicator of the efficiency of azotobacterization (eAZB) according to the first claim **characterized in that** the ZAR is visually identified by the obvious presence of RCS through a section of the soil surface, or implicitly on the basis of the setting of the tillage implement used for burying the RCS such as, for example, a coulter plow or again tillage implements with discs and disc derivatives.

3. Method for the formation of an elementary indicator of the efficiency of azotobacterization (eAZB) according to any one of the preceding claims, **characterized in that** the ZAR specimen for determining rNN50iv is taken in the fall immediately after the burial of the cellulosic crop residues (RCS).

4. Method for the formation of an elementary indicator of the efficiency of azotobacterization (eAZB) according to any one of the preceding claims **characterized in that** the ZAR specimen is taken repeatedly in order to determine rNN over time and to establish the kinetics thereof, advantageously daily, for example using specific microprobes for N-NO3 and N-NH4.

5. Method for the formation of an elementary indicator of the efficiency of azotobacterization (eAZB) according to any one of the preceding claims, **characterized in that** for TSL (sunflower) and ZEA (corn) RCS buried late in the fall, or again BTH (winter wheat, cereals) and CLZ (rapeseed) RCS buried earlier on at the end of summer, ZAR specimens are taken between 0 and 7 days (post-burial), and more particularly as soon as possible post-burial.

6. Method for the formation of an elementary indicator of the efficiency of azotobacterization (eAZB) according to any one of the preceding claims **characterized in that** the moment given for the establishment of rNN50iv corresponds to an accelerated rNN increase during in vitro incubation of the said ZAR specimen at constant temperature and humidity, and more particularly at the moment corresponding to the point of inflection of an empirical function with asymptotic plateaus of type;

$$rNN = a + (d - a) / [1 + (jpi / c)\, b]$$

knowing that the parameters *a* and *d* are asymptotic parameters (a being the minimum and *d* the maximum), *b* is the slope parameter, the parameter *c* corresponds to the abscissa of the mid-slope point, or point of inflection, whose ordinate is (*a* + *b*) / 2 when rNN = rNN50iv, and jpi (days post incubation initiation) the period of time, in days, elapsed between the start and the end of the said *in vitro* incubation of the ZAR specimen.

7. Method for the formation of an elementary indicator of the efficiency of azotobacterization (eAZB) according to claims 1 and 2, **characterized in that** the ZAR for determining rNN50is is taken early spring at winter's end.

8. Method for the formation of an elementary indicator of the efficiency of azotobacterization (eAZB) according to the preceding claim **characterized in that** the ZAR specimen is taken punctually - intermittently - and repeatedly in order to obtain a representative and discrete measurement of rNN in this sample.

9. Method for the formation of an elementary indicator of the efficiency of azotobacterization (eAZB) according to any one of the two preceding claims **characterized in that** the index rNN50is is obtained directly from an empirical function describing rNN50is according to the value of rNN at the time the aforementioned specimens are taken (jpe *), jpe * being the jpe at which the relation rNN50is :: rNN is the strongest, and more particularly using a logistic function with asymptotes with four parameters;

$$rNN50 = a + (d - a) / [1 + (rNNjpe * / c)\, b] = rNN50is$$

knowing that the parameters *a* and *d* are asymptotic parameters (a being the minimum and *d* the maximum), *b* the slope parameter, and *c* the parameter corresponding to the abscissa of the mid-slope point whose ordinate is defined as (*a* + *b*) / 2 when rNN = rNN50is.

10. Method for the formation of an elementary indicator of the efficiency of azotobacterization (eAZB) according to any one of the three preceding claims, **characterized in that** the moment at which the ZAR specimen is taken - number of days - the most indicative of rNN as an index of the contribution of Nm (jpe *) is - for the TSL and ZEA like RCSs buried late in the fall - between 50 and 70 jpe, and more particularly at 60 jpe; for BTH and CLZ like RCSs buried more hastily at the end of summer, this moment in time is between 120 and 160 jpe, and more particularly at around 150 jpe.

11. Method for the formation of an elementary indicator of the efficiency of azotobacterization (eAZB) according to any one of the preceding claims, **characterized in that** the values of rNN50iv and/or rNN50is as correlated with eAZB - i.e. efficiency of the azotobacterization of the RCS in agronomic terms such as grain or protein yields when compared to non-azotobacterized RCS controls are used as an index predicting eAZB on the following crop established onto these buried RCS.

y = 0.4976x + 9.908
R² = 0.7181

y = 0.2725x + 29.596
R² = 0.3387

RSH 0-90 cm (kgN/ha)

RAT 0-30 cm (kgN/ha)

Figure 1

Figure 2

Figure 3

Figure 4

Figure 5

Figure 6

Figure 7

Figure 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 17196251
- FR 2833016
- EP 2314669 A
- EP 2684588 A
- US 2013046468 A1
- EP 2819498 A1
- EP 2942621 A
- EP 2213154 A
- EP 13366004

**Littérature non-brevet citée dans la description**

- **ABRAS, J-P GOFFART ; J-P DESTAIN.** Perspectives d'amélioration du conseil prévisionnel de fertilisation azotée à la parcelle en Wallonie par l'utilisation du logiciel AzoFert®. *Biotechnol. Agron. Soc. Environ.,* 2013, vol. 17 (S1), 215-220
- **BENDIKOV, TA. ; J KIM ; TC. HARMON.** Development and environmental application of a nitrate selective microsensor based on doped polypyrrale films. *Sensors and Actuators B,* 2005, vol. 106, 512-517
- Conceptual basis, formalisations and parameterization of the STICS crop model. Éds. Quae (Inra). 2008
- **CAMBARDELLA CA ; TB MOORMAN ; JM NOVAK ; TB PARKIN ; DL KARLEN ; RF TURCO ; AE KONOPKA.** Field scale variability of soil properties in central Iowa soils. *Soil Sci. Soc. Am. J.,* 1985, vol. 58, 1501-1511
- **CLAUDE, P.-P. ; MACKENZIE, A. F. ; COULMAN, B. E. ; MEHUYS, G. R.** Effet du labour printanier et des cultures intercalaires de trbile rouge sur le rendement du maïs-grain. *Can. J. Plant Sci.,* 1993, vol. 73, 37-46, http://www.nrcresearchpress.com/doi/pdfplus/10.4141/cjps93-006
- **CLAUDE, P-P ; M. GIROUX.** Effet des engrais organo-minéraux inoculés (EOMI) sur la croissance des plants de maïs-grain, les rendements, les prélèvements des éléments nutritifs et la qualité des grains. *Agrosol,* 2006, vol. 17, 51-64, https://www.irda.qc.ca/assets/documents/Publications/documents/claude-giroux-2006 article effet eomi mais.pdf
- **CLAUDE, P.-P. ; L. FILLION.** Effet de l'apport d'un inoculum bactérien aux résidus de culture de maïs-grain au sol sur le rendement et la qualité de blés d'hiver panifiables en France. *Agrosol,* 2004, vol. 15, 23-29, http://collections.banq.qc.ca/ark:/52327/bs604022
- Calcul de la fertilisation azotée : Guide méthodologique pour l'établissement des prescriptions locales. **COMIFER.** Comifer Le Diamant A. 2013
- **CONSTANTIN, J. ; N. BEAUDOIN ; M. LAUNAY ; J. DUVAL ; B. MARY.** Long-term nitrogen dynamics in various catch crop scénarios: Test and simulations with STICS model in a temperate climate. *Agriculture, Ecosystems and Environment,* 2012, vol. 147, 36-46
- **DUMANSKI, J. ; W.W. PETTAPIECE ; D.F. ACTON ; P-P. CLAUDE.** Application of agro-ecological concepts and hierarchy theory in the design of databases for spatial and temporal characterization of land and soil. *Geoderma,* 1993, vol. 60, 343-358, http://www.sciencedirect.com/science/article/pii/001670619390035J
- **GRIGNANI, C ; L ZAVATTARO.** A survey on actual agricultural practices and their effects on the mineral nitrogen concentration of the soil solution. *Eur. J. Agron.,* 2000, vol. 12, 251-268
- **HOULÈS ; VIANNEY ; BRUNO MARY ; MARTINE GUÉRIF ; DAVID MAKOWSKI ; ERIC JUSTES.** Evaluation of the ability of the crop model STICS to recommend nitrogen fertilisation rates according to agro-environmental criteria. *Agronomie,* 2004, vol. 24, 339-349 339
- **JUSTES, E. ; B. MARY ; B. NICOLARDOT.** Quantifying and modelling C and N mineralization kinetics of catch crop residues in soil: parameterization of the residue décomposition module of STICS model for mature and non-mature residues. *Plant Soil,* 2009, vol. 325, 171-185
- **LADHA, J. K. et al.** Global nitrogen budgets in cereals: A 50-year assessment for maize, rice, and wheat production systems. *Sci. Rep.,* 2016, vol. 6, 19355

- **LAIREZ, J ; P FESCHET ; J AUBIN ; CH BOSKSTALLER ; I BOUVAREL.** Agriculture et développement durable : guide pour l'évaluation multicritère. 2015
- **LARSEN ; LARS HAUER ; NIELS PETER REVSBECH ; SVEND JØRGEN BINNERUP.** A Microsensor for Nitrate Based on Immobilized Denitrifying Bacteria. *Appl. Environ. Microbiol.,* 1996, vol. 62, 1248-1251
- **LISKA, AJ ; H YANG ; M MILNER ; S GODDARD ; H BLANCO-CANQUI ; MP PELTON ; XX FANG ; H ZHU ; AE SUYKER.** Biofuels from crop residue can reduce soil carbon and increase CO2 émissions. *Nature Climate Change,* Mai 2014, vol. 4
- **LIU, J ; JR. WILLIAMS ; X WANG ; H YANG.** Using MODAWEC to generate daily weather data for the EPIC model. *Environmental Modelling & Software,* 2009, vol. 24, 655-664
- **MANZONI, S ; A PORPORATO.** Soil carbon and nitrogen mineralization: Theory and models across scales. *Soil Biology & Biochemistry,* 2009, vol. 41, 1355-1379
- **MENGEL, K.** Minéralisation de l'azote organique et optimisation de l'apport d'engrais azoté. *C.R. Acad. Agric. France,* 1990, vol. 76 (8), 3-12
- **OKAJIMA ; HIDEO ; ICHIRO TANIYAMA.** Significance of mass flow in nitrate-nitrogen supply to plant roots. *Soil Science and Plant Nutrition,* 1980, vol. 26 (3), 363-374
- **RECOUS, S. ; D. ROBIN ; D. DARWIS ; B. MARY.** Soil inorganic N availability: effect of maize residue décomposition. *Soil Biol. Biochem.,* 1995, vol. 27 (12), 1529-1538
- **RICHTER, G.M ; RICHTER, A.J ; BEBLIK, K. SCHMALSTIEG ; O. RICHTER.** N-dynamics and nitrate leaching under rotational and continuons set-aside-a case study at the field and catchment scale. *Agriculture, Ecosystems and Environment,* 1998, vol. 68, 125-138
- **TABOR, J.A. ; A.W. WARRICK ; D.E. MYERS ; D.A. PENNINGTON.** Spatial variability of nitrate in irrigated cotton: II. Soil nitrate and correlated variables. *Soil Sci. Soc. Am. J.,* 1985, vol. 49, 390-394
- **YANG, MING ; YUNTING FANG ; DI SUN ; YUAN-LIANG SHI.** Efficiency of two nitrification inhibitors (dicyandiamide and 3, 4-dimethypyrazole phosphate) on soil nitrogen transformations and plant productivity: a meta-analysis. *Nature Scientific Reports,* 2016, vol. 6, 22075
- **YANG ; MING-ZHI ; CHING-LIANG DAI ; CHYAN-CHYI WU.** A Zinc Oxide Nanorod Ammonia Microsensor Integrated with a Readout Circuit on-a-Chip. *Sensors,* 2011, vol. 11, 11112-11121